# EUROPEAN PATENT APPLICATION

(11) **EP 2 535 046 A1**
(43) Date of publication of application: **19.12.2012**
(21) Application number: 11170446.6
(22) Date of filing: 17.06.2011
(51) Int. Cl.: A61K 31/13, A61P 31/14

(54) **Use of mecamylamine in the treatment of pathogen-infected patients with central nervous system disorder**

(71) Applicant: Institut Pasteur, 75724 Paris Cédex 15 (FR)
(72) Inventor: Chopy, Damien, Maximilien, 75015 PARIS (FR); Lafon, Monique, 75015 PARIS (FR)
(74) Representative: Desaix, Anne

(57) **Abstract**

The invention relates to mecamylamine or a composition comprising mecamylamine, for use in the therapeutic treatment of an animal or a human patient presenting with a disorder of the central nervous system, said animal or patient being suspected of having an infection by a pathogen which triggers immune unresponsiveness, or for use to restore immune response in said animal or said human patient. In a particular embodiment, the invention is directed to mecamylamine or a composition comprising mecamylamine, for use to treat established rabies in a human patient or to treat a human patient presenting with clinical symptoms of rabies.

## Description

### FIELD OF THE INVENTION

The invention relates to mecamylamine or a composition comprising mecamylamine, for use in the therapeutic treatment of an animal or a human patient presenting with a disorder of the central nervous system, said animal or patient being suspected of having an infection by a pathogen which triggers immune unresponsiveness. The invention also relates to mecamylamine or a composition comprising mecamylamine, for use to restore immune response in an animal or a human patient presenting with a disorder of the central nervous system, said animal or patient being suspected of an infection by a pathogen which triggers immune unresponsiveness.

### BACKGROUND OF THE INVENTION

Various pathogens have been shown to infect animal or human hosts, and to alter the functions of the central nervous system (CNS). In addition to lead to disorders in the CNS, some of these pathogens trigger an immune unresponsiveness, preventing the animal or human hosts from fighting the infection to recover. Unexpectedly, the CNS can be in some instances at the very origin of the immune unresponsiveness. This CNS mediated-unresponsiveness results from the normal immune-neuroendocrine functions of the CNS, which attempts to mobilize energy through the activation of the hypothalamo-pituitary-adrenal axis (HPA axis) and to restore body homeostasis through the cholinergic anti-inflammatory pathway. Activation of these pathways leads to immune-unresponsiveness and may have the side effect to reduce the capacity of the infected host to fight the infection leading eventually to the death of the animal or human host.

One of these pathogens, the RABV virus, is a negative strand RNA virus belonging to the *Rhabdoviridae* family, transmitted by the bite of an infected animal, that causes 55,000 human fatal encephalomyelitis per year. RABV infects mainly neurons and exploits the molecular machinery of the infected neurons to migrate and progress in the Nervous System (NS) network to reach the spinal cord, the brain and finally the salivary glands. RABV virulence relies on several factors such as its capacity to avoid premature death of infected neurons and on its property to escape the immune response by several sophisticated strategies (Lafon, 2011). In addition, RABV infection is accompanied by an immune-unresponsiveness (Camelo *et al,* 2001; Hirai *et al,* 1992; Kasempimolporn *et al,* 1997; Kasempimolporn *et al*, 2001; Perry *et al,* 1990; Torres-Anjel *et al,* 1988; Tshikuka *et al,* 1992; Wiktor *et al,* 1977a; Wiktor *et al,* 1977b). This immune unresponsiveness which appears when the brain is infected is characterized by the impairment of T cells functions with an alteration of cytokine pattern, an inhibition of T cells proliferation and the destruction of immune cells without modifying immune cells proportion (CD4/CD8 ratio constant) in the lymphoid organs (Perry *et al,* 1990). This leads to the atrophy of the spleen and the thymus of RABV infected mammals. TNF-alpha receptor has been found to play a role in RABV immune-unresponsiveness, since immune cells lacking the TNF alpha p55 receptor were less immunosuppressed compared to wild type (WT) (Camelo *et al*, 2000). Infection of the brain is required, since immune unresponsiveness does not occur after the infection of the CNS with a RABV strain which infects the spinal cord only and causes only abortive disease (Camelo *et al,* 2001). HPA axis has been suspected to play a role in this process since mice strain having a less efficient HPA axis happen to be less susceptible to rabies (Roy and Hooper, 2007). Resistance to the disease can be reversed in this strain of mice by a corticosteroid treatment indicating that RABV induced immune unresponsiveness involves the HPA axis. HPA pathway is one of the two main immune-neuroendocrine pathways executed by the NS to modulate immune function. Stimulation of the HPA axis leads to glucocorticoids release by the adrenal glands. Glucocorticoids have anti-inflammatory effects by preventing the expression of proinflammatory cytokines (TNF-alpha, IL-1), chemokines (CCL2, CXCL10), prostaglandins (COX-2) or inducible nitric oxide (iNOS) (Szabo *et al,* 1994; Tait *et al*, 2008). Glucocorticoids also decrease the ability of monocytes and dendritic cells to present antigen to T cells, slow down immune cells proliferation and induce apoptosis in leukocytes and T lymphocytes (Smets *et al,* 1999). During infectious diseases such as influenza virus infection, it has been proposed that glucocorticoid production through the HPA axis activation compromises the innate immune response and favours a secondary bacterial infection (Jamieson *et al,* 2010). Similar neuro-immuno-modulatory pathway can be activated during neurotropic virus infection such as infection by herpes virus, cytomegalovirus and New Castle disease virus (Camelo *et al,* 2001; Roy and Hooper, 2007; Silverman *et al,* 2005).

However, many uncertainties remain regarding the mechanism of immune unresponsiveness triggered during RABV infection. In particular, adrenalectomy (removal of adrenal glands, the main producers of glucocorticoids in the body) is not sufficient to abrogate immune unresponsiveness (Perry *et al,* 1990) in RABV-infected mice, suggesting that others pathways unidentified yet are probably involved in the progression of the disease.

Finally, therapeutic options are still insufficient to fight the rabies infection, since despite that preventive and prophylactic vaccines can efficiently protect against rabies if it is promptly applied before the appearance of the first clinical symptoms, no treatment is efficient once RABV has reached the brain.

Therefore, there still exists a need in the art for therapeutic molecules that can be used in various stages of infection by pathogens triggering immune unresponsiveness, molecules which can be identified following a better understanding of the mechanisms involved in the progression of the pathogen and in the implementation of the immune unresponsiveness.

### BRIEF DESCRIPTION OF THE DRAWINGS

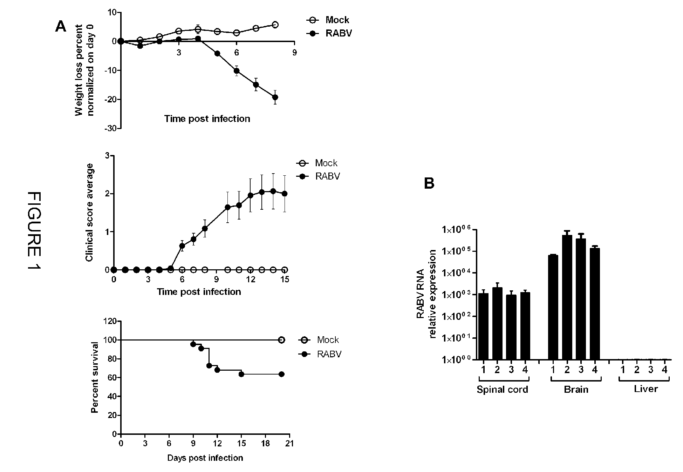

**Figure 1****. RABV causes fatal encephalitis in C57BI6 mice and triggers infection of brain, spinal cord but not liver.** Mice (six week old female C57BI6) were intramuscularly injected in each hind limb with a dose of an encephalitic RABV strain expected to kill 50-40% of the mice or vehicle (Mock). **(A)** Progression and outcome of RABV infection were followed by recording body weight, clinical signs (ruffled fur, hindlimb paralysis, hunchback - a sign of encephalitis- and death), and mortality (bottom panel) daily (n=8). Weight loss was expressed as mean +/-SEM of individual weight loss normalized on the weight of mice at day=0 (high panel). Clinical score is the mean +/-SEM of individual clinical scores (middle panel). Mock represent negative control (non-infected or NI) mice. **(B)** Mice were sorted according to the severity of the clinical signs (1 = ruffled fur, 2 = one paralyzed hind leg, 3 = two paralyzed hind legs and hunched back, 4 = tetraplegia). The N RABV gene RNA was quantified in the spinal cord, brain and liver of RABV-infected WT mice by qRT-PCR. Results are shown as mean +/-SEM (n=3).

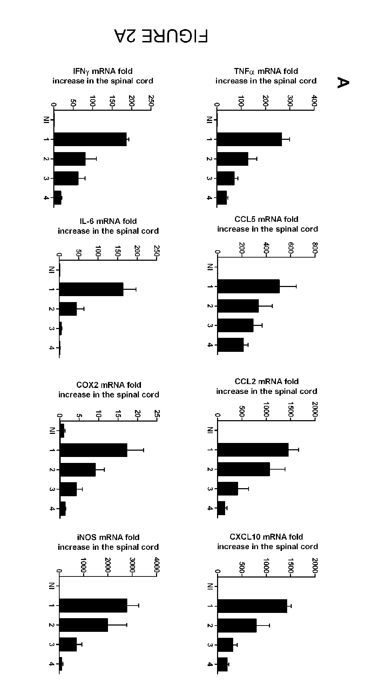

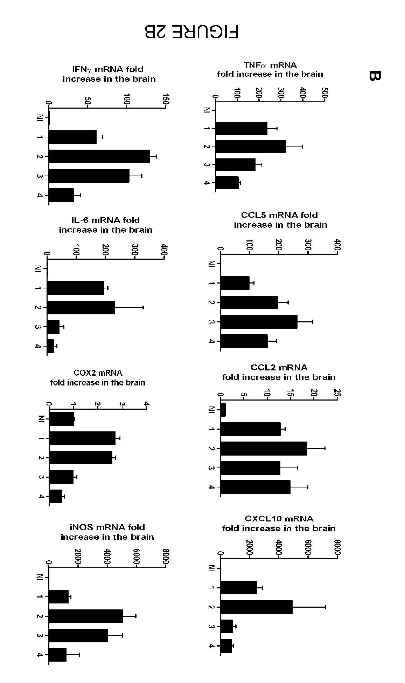

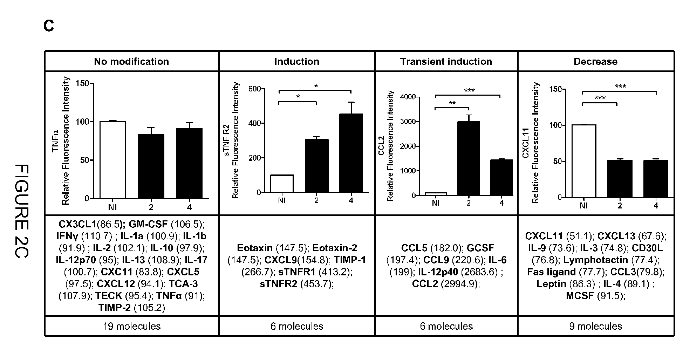

**Figure 2****. The inflammatory pattern of the spinal cord and brain in RABV-infected mice.** The relative fold increases of *TNFα, CCL5, CCL2, CXCL10, IFN*γ, *IL-6, COX2* and *iNOS* transcripts were measured by qRT-PCR in the spinal cord **(A)** and in the brain **(B)** of C57BL6 mice infected with RABV and sacrificed at different stages of the pathology (stages 1 to 4 as defined above). Data are presented as mean ± SEM (n=3) normalized on NI value set to 1. **(C)** Ab arrays measuring the expression of 40 proteins involved in inflammation were performed on NI and RABV-infected brains of mice sacrificed at two stages of RABV pathology (2 = one paralyzed hind leg, and 4 = tetraplegia) (n=4). Expression of each marker was relatively quantified based on the level of NI mouse brain set up to 100. Inflammatory markers were classified according to their profile of expression: no modification, induction, transient induction and decrease of expression compared to expression in the brain of NI mice. Graphs present the mean ± SEM of the relative expression of representative markers of each category. Numbers in brackets correspond to the mean fold variation of expression. Student's T test: * *p ≤ 0.05, p*< *0.005 and *** p ≤ 0.0005.*

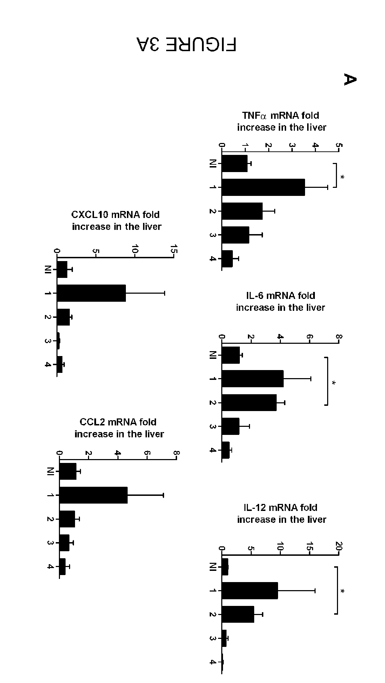

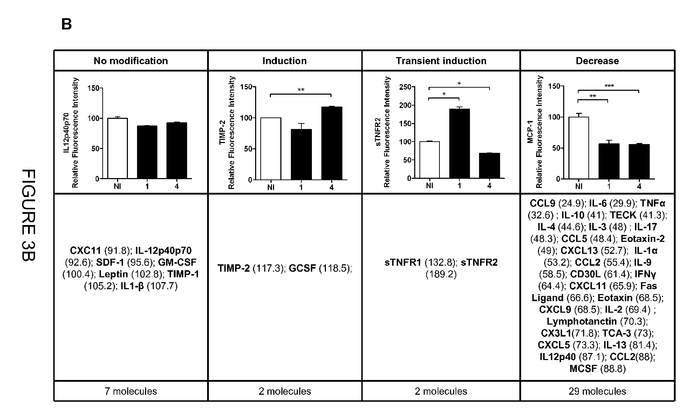

**Figure 3****. The inflammatory pattern of the liver in RABV-infected mice. (A)** The relative fold increase of *TNFα, IL-6, IL-12, CXCL10* and *CCL2* transcripts were measured by qRT-PCR in the liver of C57BL6 mice infected with RABV and sacrificed at different stages of the pathology (stages 1 to 4 as defined above). Data are presented as mean ± SEM (n =3). **(B)** Ab arrays measuring the expression of 40 proteins involved in inflammation were performed on the liver extracts of NI and RABV-infected mice sacrificed at two stages of pathology (1 = ruffled fur and 4 = tetraplegia) (n=2). Expression of each marker was relatively quantified based on the level of NI liver extract set up to 100. Inflammation markers were classified according to their expression profile: no modification, induction, transient induction and decrease of expression compared to NI. Graphs present the mean ± SEM of the relative expression of representative markers in each category. Numbers in brackets correspond to the mean fold range variation. Student's T test: * *p ≤* 0.05; ** *p* ≤ 0.005; *** *p* ≤ 0.0005.

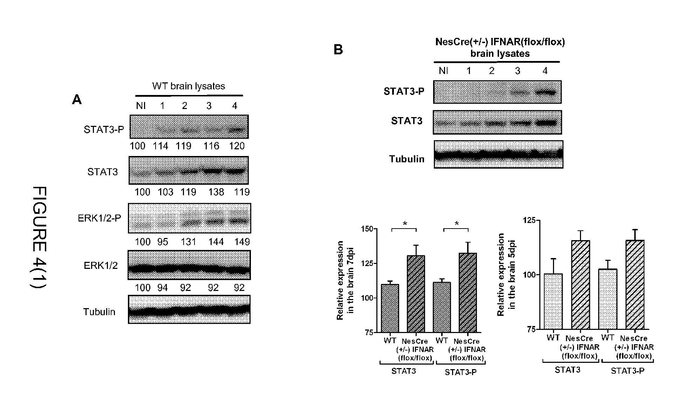

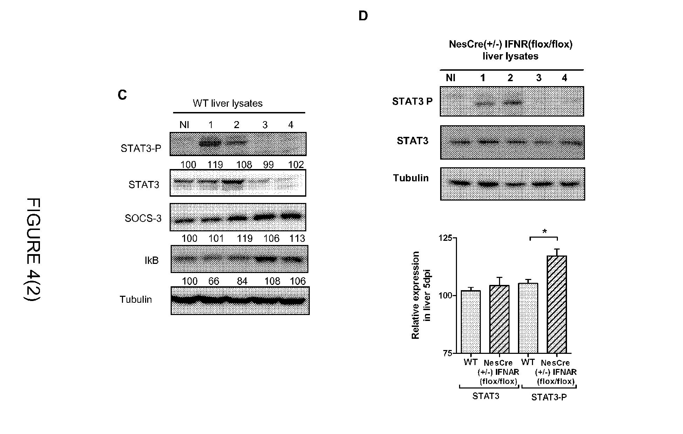

**Figure 4****. Markers of the cholinergic anti-inflammatory pathway are expressed in the liver and the brain during RABV infection. (A) STAT3,** phosphorylated-STAT3 (Tyr705), STAT3-P, SOCS-3, IkB and alpha-tubulin (used as loading control) expressions were measured by western blotting in the liver extracts of NI and of RABV-infected mice sacrificed at different stages of pathology (stages 1 to 4 as defined above). **(B)** STAT3, STAT3-P and α-tubulin expressions were measured in the liver extracts of NesCre(^{+/-})IFNAR(^{flox/flox}) mice infected with RABV and sacrificed at different stages of pathology (1 = ruffled fur, 2 = one paralyzed hind leg, 3 = two paralyzed hind legs and hunched back, 4 = tetraplegia) (left panel). STAT3 and STAT3-P relative expression were compared by western blotting in the liver extracts of WT and NesCre(^{+/-})IFNAR(^{flox/flox}) mice 5 days pi (post infection) (middle panel) or 7 days pi (right panel) (n=3). (C) STAT3, STAT3-P, ERK1/2, phosphorylated-ERK1/2 (Thr202/Tyr204) and alpha-tubulin were measured by western blotting in brain of NI or RABV infected mice (stages 1 to 4 as defined above). **(D)** NesCre(^{+/-})IFNAR(^{flox/flox}) were infected with RABV and sacrificed at different stages of the pathology (left panel), 5d pi (right panel) to measure STAT3, STAT3-P and α-tubulin expressions in their brain (n=3). Data are presented as mean ± SEM of the relative band intensity measured by GeneSnap Tool software. Student's T test :* *p* ≤ 0.05.

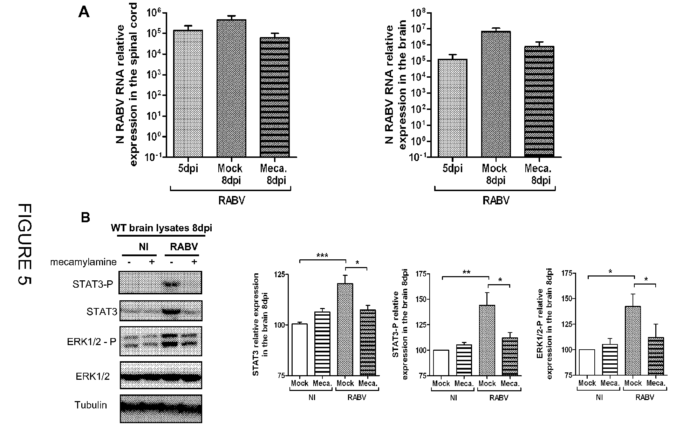

**Figure 5****. Treatment with mecamylamine, a non-competitive acetylcholine receptor antagonist drastically decreases markers of cholinergic activation in the brain.** Non-infected (NI) and RABV-infected mice were injected with mecamylamine (2mg/kg, ip) or vehicle (mock) 5 days pi and sacrificed 8 days pi (n=8). **(A)** N RABV gene expression was compared in the spinal cord and brain of NI and RABV-infected mice, either mock-treated or treated with mecamylamine 5 days pi using qRT-PCR. **(B)** STAT3, STAT3-P, ERK1/2, phosphorylated-ERK1/2 (Thr202/Tyr204) and alpha-tubulin were measured by western blotting in brain extracts from mice mock-treated or treated with mecamylamine) 5 days pi and sacrificed 8 days pi. Assays were performed on treated and non-treated mice having similar N RABV transcript in other half brain 8 dpi (n=3). Data are presented as mean ± SEM of the relative band intensity measured by GeneSnap Tool software. Student's T test: ** p* ≤ 0.05, ** *p* ≤ 0.005, ***p<= 0.0005.

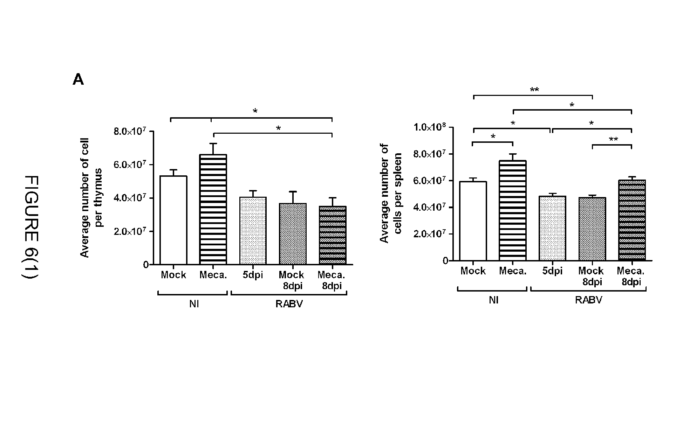

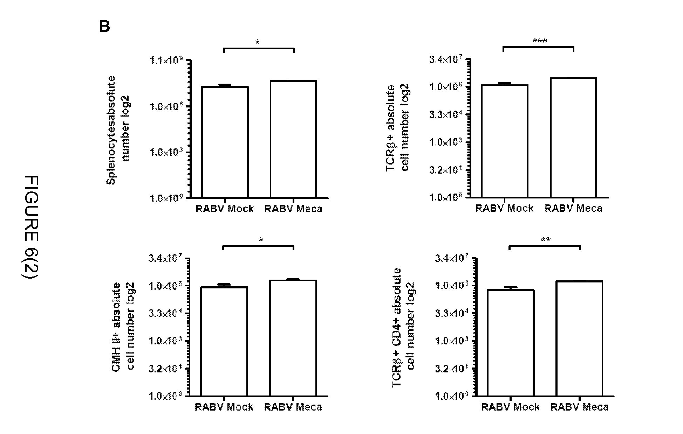

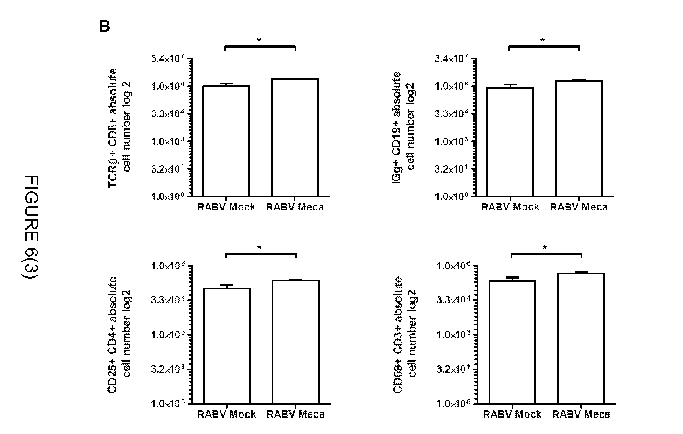

**Figure 6****. Mecamylamine prevents RABV-mediated spleen atrophy and disappearance of specific subsets of immune cells. (A)** NI and RABV infected mice were treated with mecamylamine 5 days pi (2mg/kg, ip) or not treated (mock). Cell numbers in the thymus (left panel) and the spleen (right panel) (n=3-7) were counted 5 and 8 days pi. Data are presented as mean ± SEM (* *p* ≤ 0.05; ** *p ≤* 0.005; *** *p ≤* 0.005). **(B)** Mecamylamine prevents disappearance of main splenocytes subsets in day 10 RABV-infected mice. N=6 mice per group. Data are presented as mean ± SEM (* *p* ≤ 0.05; ** *p* ≤ 0.005; *** *p* ≤ 0.005).

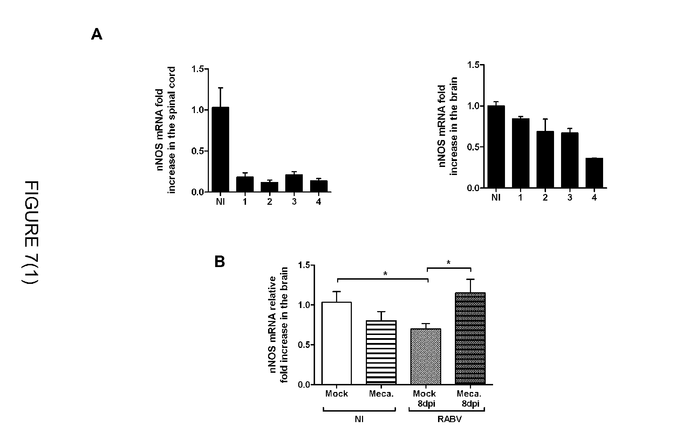

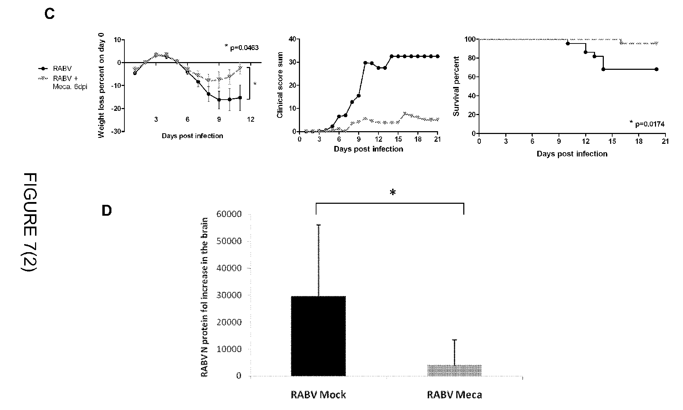

**Figure 7****. Mecamylamine maintains nNOS brain expression, triggers viral clearance off the brain and protects mice against rabies death. (A)** Transcripts of the neuroprotective molecule nNOS were measured by qRT-PCR in the spinal cord (left panel) and the brain (right panel) of mice infected with RABV and sacrificed at different stages of the pathology (stages 1 to 4 as defined above) (n=3). **(B)** nNOS transcripts were measured by qRT-PCR in the brain of RABV infected mock-treated or treated with mecamylamine 5 days pi and sacrificed 8 days pi. Data are presented as mean ± SEM (n =3). Student's T test: * *p* ≤ 0.05. **(C)** RABV pathogenesis was compared in C57BI6 mice either mock-treated or inoculated with mecamylamine (2mg/kg ip) 6 days pi by monitoring weight loss (upper right panel) (n=10 per group), clinical scores (upper left panel) (n=22 per group) and survival rate (lower left panel) (n=22 per group). **(D)** N RABV gene expression was compared in the spinal cord and brain of NI- and RABV-infected mice, either mock-treated or treated with mecamylamine 20 days pi using qRT-PCR. Data are presented as mean of relative fold increase of transcripts ± SEM (* *p* ≤ 0.05). NI value is taken as 1.

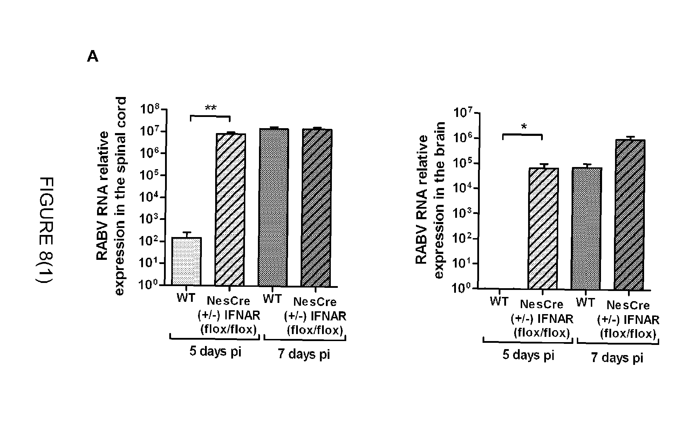

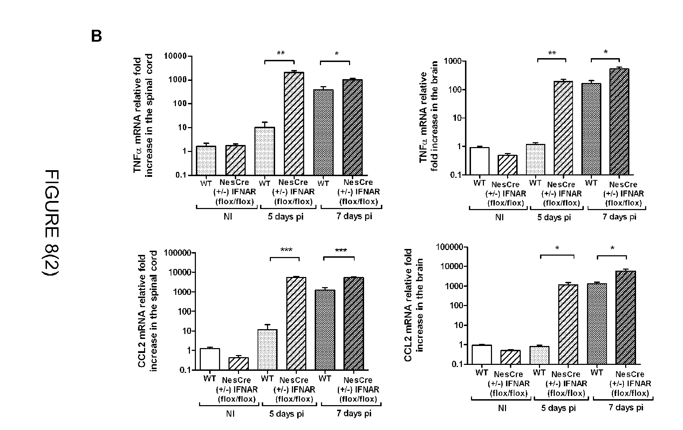

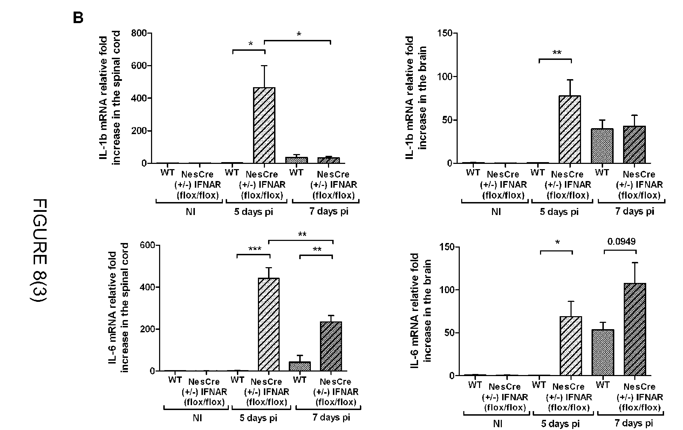

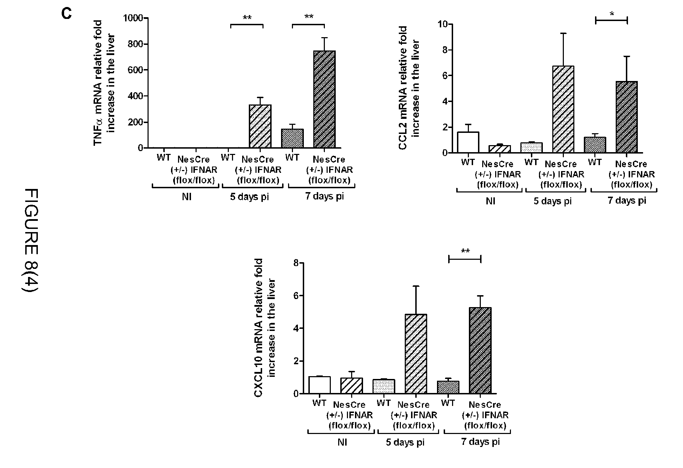

**Figure 8****. The NS inflammation and TNFα, IL-6 and CXCL10 induction in the liver correlates with NS infection. (A)** N RABV gene expression was measured by qRT-PCR, 5 and 7 days pi with RABV, in the spinal cord (left panel) and the brain (right panel) of WT and NesCre(^{+/-})IFNAR(^{flox/flox}) mice. **(B)** *TNF*α, *CCL2, IL1-β*, *IL-6* transcriptions were measured using qRT-PCR in the spinal cord (left column) and the brain (right column) of WT mice and NesCre (^{+/-}) IFNAR (^{flox/flox}) mice sacrificed 5 and 7 days post i.m. (intra muscularly) inoculation with RABV (n=5). **(C)** *TNFα, CCL2,* and *CXCL10* gene transcriptions were measured using qRT-PCR in the liver (left column) of WT and NesCre (^{+/-}) IFNAR (^{flox/flox}) sacrificed 5 and 7 days post i.m. inoculation with RABV (n=5). Data are presented as mean ± SEM (* *p* ≤ 0.05; ** *p* ≤ 0.005; *** *p* ≤ 0.005).

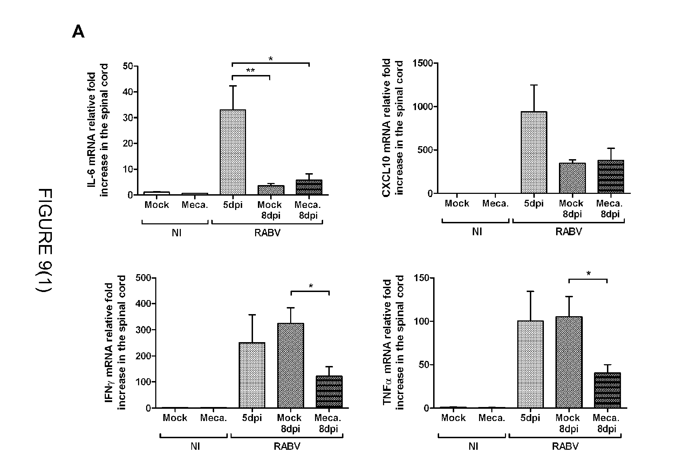

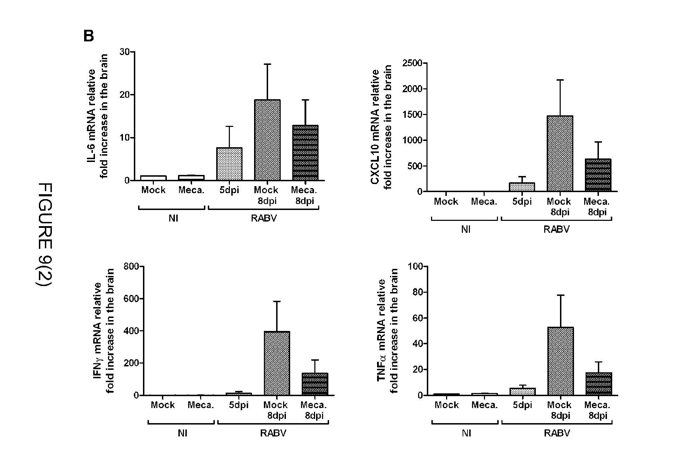

**Figure 9****. Mecamylamine does not modify the inflammatory pattern in the brain.** Non infected and RABV infected mice were injected with mecamylamine (2mg/kg ip) or vehicle (mock) 5 days pi and sacrificed 8 days pi to measure *IL-6*, *CXCL10, IFN-γ* and *TNF*α in their spinal cord **(A)** and their brain **(B)** using qRT-PCR (n=8). Data are presented as mean ± SEM. (* *p* ≤ 0.05; ** *p* ≤ 0.005).

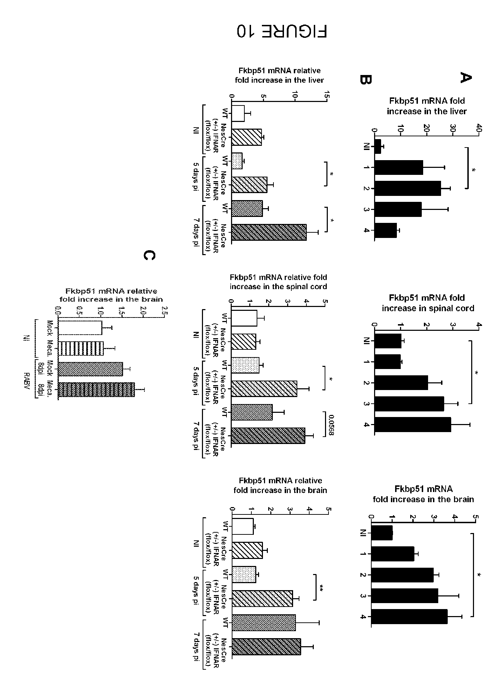

**Figure 10****. Fkbp51 transcription, a glucocorticoid inducible gene, is induced in the liver, the spinal cord and the brain of RABV infected mice. (A)** WT mice i.m infected with RABV were sacrificed at different stages of the pathology (stages 1 to 4 as defined above) to monitor *Fkbp51* (a glucocorticoid inducible gene expression in their liver (left panel), their spinal cord (middle panel) and their brain (right panel) using qRT-PCR. **(B)** *Fkbp51* transcription was compared using qRT-PCR in the liver (left column), the spinal cord (middle panel) and the brain (right column) of WT mice and NesCre ^{(+/-)} IFNAR ^{(flox/flox)} mice sacrificed 5 and 7 days post i.m. inoculation with RABV (n=5). Data are presented as mean ± SEM (* *p ≤* 0.05; ** *p* ≤ 0.005). **(C)** Effect of mecamylamine treatment on *Fkbp51* transcription in the brain of RABV infected treated or mock treated with mecamylamine and sacrificed 8 days pi. Data are presented as mean ± SEM (n =3).

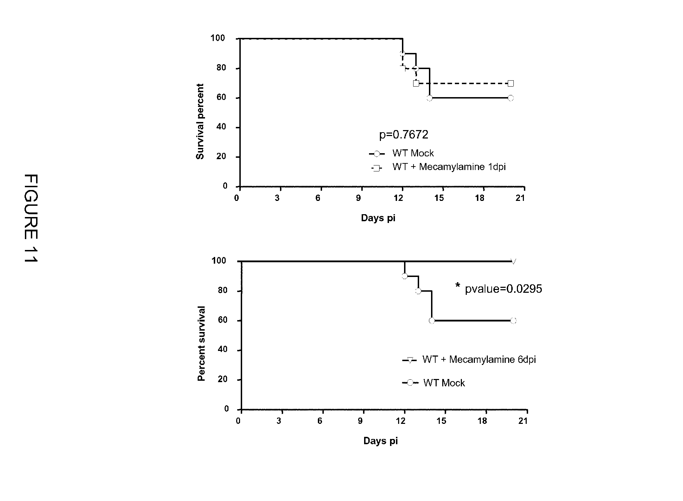

**Figure 11****. Mecamylamine has no protective effect on RABV pathogenicitity when injected early after infection (day +1 pi).** Survival curves of RABV-infected C57BI6 6 week old female mice injected with mecamylamine (2mg/kg, ip) or vehicle (mock) either 1 day (left panel) or 6 days after infection. (n=8).

### DETAILED DESCRIPTION

The invention relates to mecamylamine or a composition comprising mecamylamine, for use in the therapeutic treatment of an animal or a human patient presenting with a disorder of the CNS, said animal or patient being suspected of having an infection by a pathogen which triggers immune unresponsiveness. The invention also relates to mecamylamine or a composition comprising mecamylamine, for use to restore immune response in an animal or human patient presenting with a disorder of the CNS, said animal or patient being suspected of having an infection by a pathogen which triggers immune unresponsiveness.

The invention is directed to a method for the therapeutic treatment of an animal or a human patient presenting with a disorder of the CNS, said animal or patient being suspected of having an infection by a pathogen which triggers immune unresponsiveness, comprising (1) the administration of mecamylamine or a composition comprising mecamylamine into said animal or human patient, and (b) the follow up of the condition of said animal or human patient.

The invention is also directed to a method to restore immune response in an animal or a human patient presenting with a disorder of the CNS, said animal or patient being suspected of an infection by a pathogen which triggers immune unresponsiveness, comprising (1) the administration of mecamylamine or a composition comprising mecamylamine into said animal or human patient, and (b) the follow up of the condition of said animal or human patient.

The invention concerns the use of mecamylamine or a composition comprising mecamylamine for the manufacture of a drug suitable for the therapeutic treatment of an animal or a human patient presenting with a disorder of the CNS, said animal or human patient being suspected of having an infection by a pathogen which triggers immune unresponsiveness. The invention also concerns the use of mecamylamine or a composition comprising mecamylamine for the manufacture of a drug suitable to restore immune response in an animal or a human patient presenting with a disorder of the CNS, said animal or patient being suspected of having an infection by a pathogen which triggers immune unresponsiveness.

The invention also relates to a method to manufacture a drug suitable for the therapeutic treatment of an animal or a human patient presenting with a disorder of the CNS, said animal or patient being suspected of having an infection by a pathogen which triggers immune unresponsiveness, comprising (a) formulating mecamylamine with pharmaceutically acceptable inactive ingredients and optionally with other active ingredients; and (b) recovering the administrable drug. The invention relates to a method to manufacture a drug to restore immune response in an animal or a human patient presenting with a disorder of the CNS, said animal or patient being suspected of having an infection by a pathogen which triggers immune unresponsiveness, comprising (a) formulating mecamylamine with pharmaceutically acceptable inactive ingredients and optionally with other active ingredients; and (b) recovering the administrable drug.

The invention is also directed to a composition, preferably a pharmaceutical composition, for the therapeutic treatment of an animal or a human patient presenting with a disorder of the CNS, said animal or patient being suspected of having an infection by a pathogen which triggers immune unresponsiveness, comprising mecamylamine or a composition comprising mecamylamine as an active component, especially as an effective component. This composition is also suitable to restore immune response in an animal or human patient presenting with a disorder of the CNS, said animal or patient being suspected of having an infection by a pathogen which triggers immune unresponsiveness.

The mecamylamine or the composition comprising mecamylamine is used in an animal or a human patient, presenting with a disorder of the CNS, said animal or patient being suspected of having an infection by a pathogen which triggers immune unresponsiveness. Thus, before administering mecamylamine or a composition comprising mecamylamine, the observation of clinical symptoms putting in evidence disorder(s) of the CNS is generally carried out. This step, carried out by the medical staff, is implemented using any conventional means, such as the clinical examination of the animal or the human patient to search for medical signs or symptoms, test results and if needed radiology diagnosis.

The expression "*disorders of the CNS*" means any alteration of the CNS, in particular any alteration of the brain giving rise to encephalitis or encephalopathy, which can be determined by clinical symptoms. Non exhaustive symptoms that can be used to put in evidence a disorder of the CNS are confusion, drowsiness, fatigue, seizures, convulsions, tremors, hallucinations, memory problems, loss of cognitive function, subtle personality changes, inability to concentrate, lethargy, depressed consciousness or other neurological signs such as myoclonus (involuntary twitching of a muscle or group of muscles), asterixis (abrupt loss of muscle tone, quickly restored), nystagmus (rapid, involuntary eye movement) and respiratory abnormalities, apneustic respirations, and post-hypercapnic apnea.

The expression "*being suspected of having an infection by a pathogen*" means that some factors taken together, which may be the direct detection and/or the direct identification of the pathogen involved in the CNS disorder(s), or rather may be the factors which do not represent such direct detection or identification, but are nevertheless sufficient to consider that it is highly probable that the animal or human patient is infected by a pathogen. Among these factors are the classical symptoms of infection (fatigue, malaise, weight loss, fever, night sweats and chills, vague body aches, pain, body aches), the personal history of the patient (trip in endemic country, contact with or bite by an animal suspected to be infected, contact with a diseased person, etc) as well as the clinical symptoms associated with CNS disorders that may be obviously be attributed to other causes. Thus, the suspicion of an infection by a pathogen which leads to CNS disorder(s) can be sufficient to consider that the administration of mecamylamine or of a composition comprising mecamylamine is necessary or appropriate.

According to the invention, it is considered that the disorder(s) of the CNS is (are) consecutive to the infection by a pathogen, i.e., the disorder(s) of the CNS arise(s) as a consequence of the infection by a pathogen as defined herein, even if the pathogen cannot be detected or is not detected when clinical observation and circumstances as disclosed above favour the likelihood that exposure to and infection by the pathogen have occurred.

Indeed, the absence of direct detection and/or direct identification of the pathogen in an animal or a human patient presenting with disorder(s) of the CNS may be explained by the health state of the animal or human patient (advanced stage of the disorder or infection), by the absence of detectable level of pathogen antigens or antibodies specific of this pathogen in the body fluids obtained from the animal or human patient, or by the unavailability of material enabling the detection or identification assay in some countries or areas. Such a situation is especially expected when the pathogen is a rabies-virus.

If possible, the direct detection and/or direct identification of the pathogen responsible for the infection, in particular either in the animal to be treated or in a reservoir animal, may be put in evidence using conventional techniques, such as detection of antigen or antibodies by ELISA, amplification of nucleic acids by PCR, microscopy on biopsy, performed on biological fluids or tissues, such as blood or serum samples.

In contrast, mecamylamine or a composition comprising mecamylamine are not used within the frame of the invention for CNS disorders which are not regarded as being a consequence of a pathogen infection. In a particular embodiment, it is excluded that mecamylamine or the composition comprising mecamylamine be used for traumatic brain injury following physical trauma or head injury, as well as for, autoimmune or hereditary, degenerative diseases.

By the term "*pathogen*", it is meant any agent which is the cause of the infection and, directly or indirectly, the cause of disorder(s) of the CNS in the animal or human patient. In a particular embodiment of the invention, the pathogen is a virus, including a product thereof having infection properties.

In a particular embodiment, the pathogen, preferably a virus, is neuroinvasive, i.e., it is able to enter and/or to infect the CNS. A particular subset of these neuroinvasive pathogens, consisting in neurotropic pathogens which enter and/or infect neurons, is also within the invention.

In another embodiment, the pathogen, preferably a virus, is neurovirulent, i.e., that it causes disease or disorder within the central nervous system.

In a more particular embodiment, the pathogen, preferably a virus, is both neuroinvasive and neurovirulent.

In an embodiment of the invention, the pathogen, preferably a virus, triggers an immune unresponsiveness, and is accordingly said immunosuppressive. In particular these pathogens, target a reaction of the CNS leading to a systemic immune unresponsiveness. This immune unresponsiveness reduces host capacity to fight the infection and favours pathogen infection, and eventually leading to a fatal issue for the infected host. In a particular embodiment, these pathogens triggers a CNS mediated immune unresponsiveness. "*Immune unresponsiveness*" can be determined, by quantitating immune cells and cytokines in biological samples. Thus, immune unresponsiveness is characterized by one or several of the following features: impairment of T cell function, alteration of cytokine pattern, inhibition of T cell proliferation and destruction of immune cells, with or without modifying cell proportion. In a preferred embodiment, the ratio of CD4/CD8 cells is constant. In an infected animal, such measures can be made by phenotyping the splenocytes using classical immunocytochemistry and flow cytometry methods (Camelo *et al,* 2001). In human patients, peripheral blood lymphocytes (PBL) can be collected in blood samples and cultivated. Cytokines production and proliferation of PBL, in response to different specific (RABV vaccine) or non-specific (lectins, ...) agents, can be measured by ELISA or flow cytometry (*Martinez-Arends*, 1995).

Among the immunosuppressive pathogens, immunosuppressive viruses, particularly RNA immunosuppressive viruses, are preferred. RNA viruses may be single-stranded positive-sense nonsegmented RNA viruses, single-stranded negative-sense nonsegmented RNA viruses, single-stranded negative-sense segmented RNA viruses or single-stranded positive-sense RNA viruses.

As shown in the present application, the targeted immune responsiveness is the one mediated by the cholinergic anti-inflammatory pathway, which is in particular triggered by the autonomous NS (parasympathic fibers of vagus nerves) to reduce inflammation.

Therefore, in a preferred embodiment of the invention, the pathogen involved in a CNS-mediated immune unresponsiveness activates the cholinergic anti-inflammatory pathway. Indeed, whereas activation of this pathway has already been observed by others in patients with inflammatory diseases or patients with NS injury, the present invention provides, for the first time, evidence that this pathway is involved in the immune unresponsiveness in patients infected by pathogens, preferably by viruses. Accordingly, when using mecamylamine, the invention provides an active ingredient for use in interfering with the activation of cholinergic anti-inflammatory pathway and/or for use as an antagonist of said pathway.

Thus, the activation of this cholinergic anti-inflammatory pathway has been proved to be beneficial in patients with inflammatory diseases such as pancreatic or sepsis (Pavlov and Tracey, 2006; Tracey and Abraham, 1999; van Westerloo *et al,* 2006). In contrast, in patients with NS injury (neuro-trauma or stroke), the activation of this pathway, and others neuro-immuno-modulatory pathways such as the HPA axis, expose patients to a higher risk to develop infectious diseases that largely contribute to secondary fatal casualties of NS injuries (Meisel *et al,* 2005). The cholinergic anti-inflammatory reflex is activated after the brain senses the presence of inflammatory cytokines such as TNF-alpha, IL-1β or IL-6 in the periphery, by neuronal (mainly through local afferent fibres of the vagus nerve) and by humoral pathway (Johnston and Webster, 2009). This input is processed by the CNS in frontal, hypothalamus and brainstem centres. Information processing results in NS mediated homeostatic signals such as the delivery of acetylcholine (Ach) in the close vicinity of macrophages by the efferent fibres of the vagus nerve in the inflamed organ. The anti-inflammatory property of Ach inhibits the cytokines production by the macrophages, leading to a NS control of peripheral inflammation (Johnston and Webster, 2009; Tracey, 2009; Ulloa, 2005). The cholinergic anti-inflammatory reflex requires the integrity of the vagus nerve, the presence of spleen and the expression of nicotinic acetylcholine receptor subunit α7 (α7nAChR) on cytokine producing cells (Tracey, 2009). The Ach signalling through α7nAChR increases intracellular calcium concentration in neurons, induces protein kinase A-dependent phosphorylation and activation of extracellular signal-regulated kinase 1 (ERK1) and ERK2 (Dajas-Bailador *et al*, 2002a; Dajas-Bailador *et al,* 2002b). Engagement of the α7nAChR at the surface of monocytes and macrophages stimulates the Janus kinase 2 (JAK2), phosphorylates and activates the signal transducer and activator of transcription 3 (STAT3) that impairs NF-κB binding to DNA and in turn prevents the inflammatory cytokines expression (Pena *et al,* 2010). The α7nAChR signalling also inhibits the phosphorylation and the degradation of IkB, the inhibitor of NF-κB and increases the expression of suppressor of cytokine signalling 3 (SOCS3) and thus inhibits inflammatory cytokines induction (de Jonge *et al,* 2005; Yoshikawa *et al,* 2006).

A particular example of pathogen which is immunosuppressive is a virus selected from the group consisting of RABV, measles virus, NewCastle disease virus, simian immunodeficiency virus (SIV) and human immunodeficiency virus (HIV).

The measles virus is a single-stranded negative-sense nonsegmented RNA virus (*Mononegavirales* order; *Paramyxoviridae* family; *Morbillivirus* genus) infecting humans.

RABV is a single-stranded negative-sense nonsegmented RNA virus (*Mononegavirales* order; *Rhabdoviridae* family; *Lyssavirus* genus) which shows both high neuroinvasiveness and high neurovirulence in humans.

The NewCastle Disease virus is particular virulent in poultry and affects many domestic and wild avian species. It is a single-stranded negative-sense nonsegmented RNA virus (*Mononegavirales* order; *Paramyxoviridae* family; *Avulavirus* genus).

The SIV virus and HIV virus are single-stranded positive-sense RNA retroviruses (*Retroviridae* family; *Lentivirus* genus) infecting respectively macaques and human. The HIV virus includes HIV-1 virus and HIV-2 virus.

The mecamylamine, is known as N,2,3,3-Tetramethylbicyclo[2.2.1] heptan-2 amine and has the following formula:

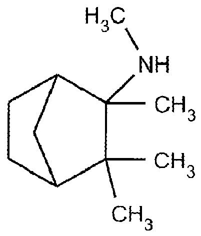

The word "*mecamylamine*" encompasses any isomer, and especially the (S)-(+)-mecamylamine, the (R)-(-)-mecamylamine, any combination of the (S)-(+)-mecamylamine and (R)-(-)-mecamylamine (*e.g.*, racemic mecamylamine) and any pharmaceutically acceptable salt of mecamylamine. A particular isomer of mecamylamine is the (S)-(+)-mecamylamine. In a particular embodiment, a composition comprising the (S)-(+)-mecamylamine is free of the (R)-(-)-mecamylamine. In another embodiment, a composition comprising the (R)-(-)-mecamylamine is free of the (S)-(+)-mecamylamine. By "*free of*", it is means that the composition comprising the (S)-(+)-mecamylamine or the (R)-(-)-mecamylamine contains at least about 95% by weight of the (S)-(+)-mecamylamine or the (R)-(-)-mecamylamine respectively, and less than about 5% by weight of the (R)-(-)-mecamylamine or the (S)-(+)-mecamylamine, respectively. In particular embodiment, a composition comprising the (S)-(+)-mecamylamine or the (R)-(-)-mecamylamine contains at least about 98% or at least about 99% by weight of the (S)-(+)-mecamylamine or the (R)-(-)-mecamylamine respectively.

The synthesis of the mecamylamine is fully described in U.S. Patent 2,831,027 or in the publication of Stein, G. A. et al. (1956). Journal of the American Chemical Society 78(7): 1514. The product is also available from various manufacturers.

Pharmaceutically acceptable salts of mecamylamine are also within the invention. One of these is the mecamylamine hydrochloride (N,2,3,3-Tetramethylbicyclo[2.2.1]heptan-2-amine hydrochloride). The expression "*mecamylamine hydrochloride*" encompasses all isomers of mecamylamine hydrochloride, and especially the exo-S-mecamylamine hydrochloride and the exo-R-mecamylamine hydrochloride. A particular isomer is the exo-S-mecamylamine hydrochloride. In a particular embodiment, a composition comprising the exo-S-mecamylamine hydrochloride is free of the exo-R-mecamylamine hydrochloride. In another embodiment, a composition comprising the exo-R-mecamylamine hydrochloride is free of the exo-S-mecamylamine hydrochloride. By "*free of*", it is means that the composition comprising the exo-S-mecamylamine hydrochloride or the exo-R-mecamylamine hydrochloride contains at least about 95% by weight of the exo-S-mecamylamine hydrochloride or the exo-R-mecamylamine hydrochloride respectively, and less than about 5% by weight of the exo-R-mecamylamine hydrochloride or the exo-S-mecamylamine hydrochloride, respectively. In a particular embodiment, a composition comprising the (S)-(+)-mecamylamine or the exo-R-mecamylamine hydrochloride contains at least about 98% or at least about 99% by weight of the exo-S-mecamylamine hydrochloride or the exo-R-mecamylamine hydrochloride respectively.

In a further embodiment, the invention relates to the use of mecamylamine analogues, such as substituted mecamylamine. Examples of substituted analogues of mecamylamine are disclosed in Stone et al. (1962) J. Med. Pharm Chem 5(4); 665-90. The ability of substituted analogues of mecamylamine to interrupt the cholinergic pathway, and thus their therapeutic activity, may be routinely assayed by quantitating marker(s) of the cholinergic signalling pathway.

Indeed, the activation of the cholinergic signalling pathway is characterized by the expression of STAT3, the phosphorylation of STAT3 (STA3-P) and the phosphorylation of ERK1/2 (ERK1/2-P). In contrast, the interruption of the cholinergic signalling is characterized by the disappearance of STAT3-P and a decrease of both STAT3 and ERK1/2-P. Consequently, it is possible to identify the effect of a substituted analogue of mecamylamine, by comparing the expression of at least one marker of the cholinergic signalling in an infected animal model which has received this substituted analogue of mecamylamine (treated animal) and in an infected animal model which has not received this substituted analogue of mecamylamine (non-treated animal). As markers of the cholinergic anti-inflammatory pathway, it is preferable to carry out the quantitation in the site(s) of infection. For example, using a mice infected by the RABV (see example), the quantitation is preferably carried out in the brain. The quantitation may be implemented by all conventional means: quantitative amplification, qPCR or Northern Blot for nucleic acid quantitation, and ELISA or Western Blot for protein quantitation. Thus, a substituted analogue of mecamylamine is considered appropriate for use within the invention, when the cholinergic signalling is interrupted, i.e., when at least one marker of this signalling pathway in an infected treated animal is significantly decreased (p< 0.05) as compared to the same marker in an infected non-treated animal. To ensure the veracity of the results, the marker(s) of a non-infected non-treated animal is also assayed to check that the increase of the expression and/or of the phosphorylation of the markers in an infected non-treated animal is the result of the infection (see Figures 4 and 5 of the present application).

The therapeutic use of mecamylamine or a composition comprising mecamylamine according to the invention is implemented in any animal or human patient infected by a pathogen as defined herein. Thus, in a first embodiment, mecamylamine or a composition comprising mecamylamine is administered to a mammal, in a particular to a human patient. In another embodiment, mecamylamine or a composition comprising mecamylamine is administered to poultry. In the case of the rabies virus, mecamylamine or a composition comprising mecamylamine is used for administration to a human patient.

The use of mecamylamine or a composition comprising mecamylamine aims to restore the immune response, and finally to treat the pathogen infection (therapeutic treatment).

By "*restoration of the immune response*", it is meant that immune cells are functional and/or the quantity of immune cells and the level of cytokines reach those of a non-infected animal or non-infected human patient, i.e., are not statistically different from those of a non-infected animal or non-infected human patient. Thus, T cell function is recovered, CD4⁺ T cells proliferate, cytokines are normally produced (IFN-γ, TNF-α and IL-6). In a particular embodiment, the restoration of immune response finds its expression in splenocyte division.

In a particular embodiment, mecamylamine or a composition comprising mecamylamine is used to restore the immune function against the pathogen responsible of the infection. Thus, the restoration of the immune function against the pathogen is correlated to the lowering and/or to the clearance of the pathogen in said animal or human patient.

Within the invention, the restoration of the immune response is obtained by interfering with or interrupting the cholinergic anti-inflammatory pathway. This interruption may be checked by assaying the disappearance of STAT3-P and/or the decrease of STAT3 and/or the decrease of ERK1/2-P (see above).

By "*in the therapeutic treatmenf*", it is meant that the performed steps result in improving the clinical condition of an animal or a human patient in need thereof, who suffers from disorder(s) of the CNS and/or has been diagnosed as infected or being suspected to be infected by a pathogen. Such treatment aims at:
(1) the elimination of the pathogen, or at lowering the load of said pathogen. In a situation of viral infection, the treatment may result in a significant decrease of the viral load in the animal or human patient, and in particular in a significant decrease of the plasma viral load when a viremia is observed following infection; and/or
(2) improving the clinical status of the animal or human patient, by eliminating or lowering the symptoms associated with the CNS disorder(s), such as the ones described above; and/or
(3) improving the clinical status of the animal or human patient, by eliminating or lowering the clinical symptoms resulting from the pathogen infection; and/or
(4) in a preferred embodiment, restoring to health.

Thus, in a particular embodiment of the invention, mecamylamine or composition comprising mecamylamine is for use to therapeutically treat rabies in a human patient, or for use in the therapeutic treatment of a patient presenting with clinical symptoms of rabies.

With respect to administration, mecamylamine or a composition comprising mecamylamine are administered once the clinical symptoms, putting in evidence disorder(s) of the CNS, appear or are observed. Generally, the time of apparition of these symptoms is shifted with respect to the time of infection (incubation period). This period of incubation varies from 2 weeks to 2 years for the rabies, and may reach more than 10 years in HIV infection (last stage of AIDS).

Thus, in the particular case of an infection by the RABV, mecamylamine or a composition comprising mecamylamine is administered once the clinical symptoms of rabies are observed. For HIV infection, mecamylamine or a composition comprising mecamylamine is administered once the neurological disorders associated with AIDS appear (HIV-associated dementia).

Mecamylamine may be used as such or in a composition further comprising inactive ingredients, such as pharmaceutically suitable excipient and/or carrier and/or vehicle.

Inactive ingredients are substances that have no effect in the restoration of the immune response or in the treatment of the infection or in the treatment of the CNS disorders (as defined above). More generally, inactive ingredients do not affect the structure or any function of the body of humans or animals, and thus are inert.

A "*pharmaceutically acceptable carrier*" refers to a non-toxic solid, semisolid or liquid filler, diluent, encapsulating material or formulation auxiliary of any conventional type. A "*pharmaceutically acceptable carrier*" is non-toxic to recipients at the dosages and concentrations employed, and is compatible with other ingredients of the formulation. Suitable carriers include, but are not limited to, phosphate buffered saline solutions, distilled water, emulsions such as an oil/water emulsions, various types of wetting agents sterile solutions and the like, dextrose, glycerol, saline, ethanol, and combinations thereof. Such carriers enable the pharmaceutical compositions to be formulated as tablets, pills, dragees, capsules, liquids, gels, syrups, slurries, suspensions, and the like. Carriers for parenteral administration include aqueous solutions of dextrose, mannitol, mannose, sorbitol, saline, pure water, ethanol, glycerol, propyleneglycol, peanut oil, sesame oil, polyoxyethylene-polyoxypropylene block polymers, and the like.

A "*pharmaceutically acceptable excipient*" refers to a substance that is used as a carrier or for the manufacturing of the administrable form of mecamylamine. Suitable excipients include fillers such as sugars, including lactose, sucrose, mannitol, or sorbitol; cellulose preparations such as, for example, maize, wheat, rice, or potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate. Thus, for oral use of mecamylamine, a solid excipient can be used, optionally grinding the resulting mixture, and processing the mixture of granules, after adding suitable auxiliaries if desired, to obtain tablets or dragee cores. Examples of excipients for coating of dragee or tablet are concentrated sugar solutions which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

A particular composition used within the invention comprises or consists of mecamylamine hydrochloride as active ingredient and the following inactive ingredients: acacia powder, lactose powder, cornstarch powder, calcium phosphate dehydrate, Yellow Lake Blend (LB-282), talc, magnesium sterate and water. This composition formulated as tablet is known as Inversine®.

The composition within the invention may also further comprise, in absence or in presence of inactive ingredients as defined herein, additional active ingredient(s). In particular, these active ingredients are chosen for their therapeutic activity in the restoration of the immune response or in the treatment of the pathogen infection or in the treatment of the CNS disorder.

In a particular embodiment, the composition within the invention also comprises active ingredient(s) against the pathogen infection, such as antibiotics or antiviral drugs. Examples of antiviral drugs are ribavirin, amantadine and possibly ketamine (Lockhart *et al*, 1992).

In another particular embodiment, the composition within the invention also comprises active ingredient(s) which treat the symptoms of the CNS disorders or the symptoms of the infection. An example is the phenobarbital which is indicated for the treatment of seizures.

In another particular embodiment, the composition within the invention also comprises active ingredient(s) which protect the health state of the patient, such as anaesthetics. Examples of such active ingredients are ketamine and midazolam.

In a particular embodiment, the composition comprises mecamylamine as defined above and at least one of or several of or all these additional ingredients: ketamine, midazolam, phenobarbital, ribavirin and amantadine.

Mecamylamine or composition within the invention can be administered in an animal or human patient, via different routes: parenteral administration such as subcutaneous (s.c.), intradermal (i.d.), intramuscular (i.m.), intraperitoneal (i.p.) or intravenous (i.v.) injection, oral administration and mucosal administration especially intranasal administration or inhalation.

The quantity to be administered (dosage) depends on the animal or human patient to be treated, including considering the condition of the animal or patient, the state of immune system of the animal or human patient, the route of administration and the size/weight of the animal or patient.

Thus, mecamylamine or composition comprising mecamylamine is administered, within the invention, at an amount of mecamylamine within a range from about 0.1 and 30 mg daily, in one or several administration(s), and in particular about 0.5 mg daily, 1 mg daily, 2 mg daily, 3 mg daily, 4 mg daily, 5 mg daily, 8 mg daily, 10 mg daily, 12 mg daily, 15 mg daily, 20 mg daily or 30 mg daily. In a particular embodiment, the mecamylamine is administered in an amount ranging from about 0.001 mg/kg of the animal or patient/day to 2 mg/kg/day. The daily amount of mecamylamine is preferably about 0.002 mg/kg, about 0.005 mg/kg, about 0.01 mg/kg, about 0.02 mg/kg, about 0.03 mg/kg, about 0.04 mg/kg, about 0.05 mg/kg, about 0.1 mg/kg, about 0.2 mg/kg, about 0.5 mg/kg, about 1 mg/kg, about 1.5 mg/kg or about 2 mg/kg.

The invention also relates to mecamylamine or composition comprising mecamylamine for use, in an administration as an adjunctive therapy or as a combination therapy, said therapy encompassing a treatment active against said pathogen or against the symptoms associated with the infection or against the symptoms associated with the CNS disorder(s).

"*Adjunctive therapy*" refers to any second treatment that is used in conjunction with a first treatment to increase the chance of cure, or to increase the first treatment's efficacy. In other words, adjunctive therapy acts as an aid to the first treatment. In the present invention, mecamylamine or a composition comprising mecamylamine may be used as an adjunctive therapy to a first treatment encompassing active ingredient(s) against the pathogen and/or against the symptoms associated with the infection and/or against the symptoms associated with the CNS disorder(s). In the particular case of rabies, mecamylamine or a composition comprising mecamylamine may be used as an adjunctive therapy in patients who have not responded adequately to a first postexposure treatment consisting of either a passive immunisation by instillation of rabies neutralising antibodies or stimulation of an active immune response with rabies vaccine. In that case, the first postexposure treatment is discontinued.

Within a combination therapy, mecamylamine or a composition comprising mecamylamine may also be used for a, concomitant or successive, administration with other(s) active ingredient(s) against the pathogen and/or against the symptoms associated with the infection and/or against the symptoms associated with the CNS disorder(s).

### EXAMPLES

### I. Material and methods

***Animals.*** Female C57BL/6J mice (Janvier, France) and NesCre(^{+/-}) IFNAR(^{flox/flox}) (Detje *et al,* 2009) six to eight week-old mice were intramuscularly inoculated in both hind legs with 1 × 10⁷ infectious particles of RABV CVS-NIV, a pathogenic strain causing fatal encephalomyelitis in mice after intramuscular injection (Camelo *et al,* 2000). Disease progression was evaluated by scoring clinical signs and mortality as follows: 0 = normal mice, 1 = ruffled fur, 2 = one paralyzed hind leg, 3 = two paralyzed hind legs and hunched back, 4 = tetraplegia (defined as the total loss of mobility), and 5 = death. Data were presented as a mean clinical score, a mean weight loss normalized on the weight of each mouse at day 0 and as a survival curve. In other experiments, groups of mice (at least n = 3) were deeply anesthetized and intra cardiacally perfused with saline phosphate buffer supplemented with Ca²⁺ and Mg²⁺ (SPB-Ca²⁺Mg²⁺⁾. Organs (liver, spinal cord and brain) were collected and stored at -80°C before being processed for RNA or protein extraction. Mice were injected by the intra-peritoneal (i.p.) route with 100µl of mecamylamine (2mg/kg) diluted in sterile water or with sterile water only (mock-treated).

***Ethical statement***. Animal housing and experimental protocols followed guidelines approved by the French Ministry of Agriculture and Institut Pasteur Ethical committee. The Institut Pasteur is a member of Le Comité Régional d'Ethique pour l'Expérimentation Animale (CREEA) de l'Ile de France.

***Antibodies (Ab) and reagents**.* Anti-alpha-tubulin mouse mAb (ref CP06) was from Oncogene Research, USA Products; Anti-phosphorylated-STAT3 (Tyr705) (ref 9131) rabbit Ab, anti-ERK1/2 (p42/p44) MAPK rabbit Ab (ref 9102), anti-phosphorylated ERK1/2 (p42/p44) (Thr202/Tyr204) MAPK rabbit Ab (ref 9101) and anti-IkB-α rabbit Ab (ref 9242 or 4814) were from Cell Signaling Technology (Danvers, USA). Anti-STAT3 (clone 84/Stat3, ref 610190) mouse mAb was purchased at BD Transduction Laboratories. FITC anti TCR (ref 553170), FITC conjugated anti CD69 (ref 557392), FITC anti mouse I-A/I-E (ref 553623), Phycoerythrin (PE) conjugated anti CD4 (ref 553652), PE conjugated anti CD8a (ref 553032), PE conjugated anti CD3 molecular complex (ref 555275) and PE conjugated anti CD11b (ref 557397) Abs are from BD Biosciences. PE conjugated anti CD11b (ref 22159114) and FITC conjugated anti CD25 (ref 22150253) Abs are from Immunotools. The horseradish peroxidase-conjugated anti-rabbit IgG, Rabbit TrueBlot, was from eBioscience; The horseradish peroxidase-conjugated AffiniPure F(ab')2 Fragment Donkey anti-Mouse IgG was from Jackson ImmunoResearch Laboratories (West Grove, USA). Mecamylamine drug was from SIGMA Aldrich. Primers used for qRT-PCR were purchased from QIAGEN.

***RNA extraction, RT and qPCR**.* Total RNAs were extracted with Qiazol and RNeasy kit (QIAGEN) from the spinal cord, the half-brain or a lobe of the liver. RNA quantity and quality were monitored by spectrophotometry (NanoDrop, Labteck France). Synthesis of cDNA was performed with 1 µg RNA using SuperScript II Reverse transcriptase (Invitrogen) with oligo dT primers. Quantitative real-time PCR (qRT-PCR) was performed in duplicate using the ABI PRISM 7500 Fast Sequence Detector system (Applied Biosystem) with the GoTaq qPCR Master Mix (Promega). After normalization to RNA of 18S (used as a housekeeping gene), the relative abundance of mRNA was obtained by the ΔΔCT method, by calculating the difference in threshold cycles between the test sample and the control samples (wild type, mock-treated or non-infected cells or tissue which value is set to 1). RABV RNA quantification was normalized to 18S and a standard control.

***Ab Arrays**.* Mouse Inflammation Ab Arrays, from RayBiotech, Inc. GA, USA, were assayed with brain or liver lysates (90µg/sample). Data were acquired with an AxioVision scanner and analyzed with the Axon GenePix software according to manufacturer's recommendations. The fluorescence intensity for each spot was normalized with a positive internal control present on each slide. The fluorescence intensity for the non-infected brain was defined as a standard arbitrary value of 100.

***Immunoblotting**.* RABV- and mock-infected tissues (half-brain or a lobe of the liver) were lysed with RIPA buffer (Sigma) in presence of anti-protease (cOmplete-Ultra, Roche) and anti-phosphatase (PhosSTOP, Roche) and stored at - 20°C. After denaturation, proteins (20-25 µg) were loaded into a 4-12 % Tris-Bis SDS NuPage gel (Invitrogen) with Kaleidoscope pre-stained standards (Bio-Rad). After transfer, PVDF membrane (Hybond-P, GE HealthCare) were saturated for 1 h at room temperature in saturating buffer (SPB-0.1% Tween 0.1% and 5% Bovine Serum Albumin). Membranes were incubated with primary Ab overnight at 4°C, washed 4 times in SPB-Tween, then incubated with secondary Ab coupled to horseradish peroxidase (1h at room temperature). To detect phosphorylated proteins, SPB was replaced by Saline Tris Buffer for incubation and washing. After washing, membrane was incubated in SuperSignal West Pico Chemiluminescent (Thermoscientific). Signals were acquired with a GBOX (Syngene) and monitored by the Gene Snap (Syngene) software. Band quantification was normalized on tubulin signal and expressed as fold increase based on control condition (non-infected, non-treated mice).

***Spleen and thymus cells counting**.* The thymus and the spleen of perfused mice were collected, grinded in 1ml of DMEM and filtered through nylon gauze. After dilution in SPB Ca²⁺ Mg²⁺, cell number was determined using automated cell counter (Millipore).

***Phenotyping of the immune splenocytes subsets by flow cytometry.*** Phenotype of splenocytes was performed with appropriate pairs of Ab and assessed by flow cytometry. Data analysis was performed with Cell Quest Pro Software. Results were presented as total numbers of cells per spleen.

***Measure of RABV specific neutralizing antibodies**.* The rapid fluorescent focus inhibition test was used to assay serum samples as previously described (Smith et al, 1973). Titres are expressed as IU/ml.

***Statistical analysis**.* For comparison of groups, Student's t tests were performed using GraphPad Prism version 5.00. For the animal experiments, survival rates were compared using Mantel-Cox test from this software.

### II. Results

The inflammatory response in the periphery (liver) and in the NS at different stages of RABV pathology was monitored. Then, it was investigated whether, in addition to the HPA axis, RABV infection also activates another pathway, such as the neuro-immuno-modulatrice cholinergic pathway. Finally, it was tested whether the injection of a cholinergic antagonist drug, mecamylamine, could reverse immune-unresponsiveness and promote survival of RABV infected mice instead.

### (1) RABV infection induces a low and well-controlled inflammatory response in the NS of RABV-infected mice

Mice (C57BI6) were intramuscularly injected in each hind limb with a dose of an encephalitic RABV strain expected to kill 40-50% of the mice. The virus infection results in body weight loss as early as day 5 post infection (pi). The first clinical signs appear at day 5pi and worsened with time. Death of animals occurs between day 9 and day 15pi (Fig. 1A). RABV infects the spinal cord and brain and not the liver (Fig. 1 B).

To characterize the inflammatory response in the RABV infected NS the transcription of *TNFα, IFN*γ and *IL-6* cytokines, *CCL5, CCL2, CXCL10* chemokines and the transcription of *COX-2* and *iNOS* two markers of inflammation were measured in the spinal cord and the brain of mice sacrificed at different stages of pathology (1 = ruffled fur, 2 = one paralyzed hind leg, 3 = two paralyzed hind legs and hunched back, 4 = tetraplegia), (Fig. 2). In the spinal cord (Fig. 2A), transcription of all the markers was highly induced (range of 17 to 2 800 fold increase compared to NI) as soon as the first clinical signs appeared and declined thereafter as the clinical signs worsened. In the brain, (Fig. 2B), transcription of the markers was also induced by the infection but with delay compared to spinal cords since maximum was obtained at stage 2 of the disease instead of stage 1 in the spinal cords. Transcription of *TNFα, CXCL10, IFN*γ, *IL-6, COX-2* and *iNOS* started decreasing in the brain at stage 3, whereas *CCL5* and *CCL2* transcription remained stable.

The transcription analysis was completed by an Ab array to study the variation in the expression of cytokines, chemokines and inflammation markers in the infected brain (NI, stages 2 and 4 were compared) at a protein level (Fig 2.C). In the brain, the expression of TNF-alpha, IFN-gamma, IL1-beta and IL-10 remained unchanged at the pathology stage tested. In contrast, RABV upregulated the expression of 12 markers of inflammation out of the 40 present on the protein array. They include chemokines (CCL5, CCL2, CCL9 and CXCL9), cytokines (IL-6, IL-12), and sTNFR1 and 2, two anti-inflammatory soluble proteins interfering with the binding of TNF to its receptors. RABV infection down-regulated the expression of 9 markers of inflammation. The up regulation of six markers including IL-6, CCL2 CCL5 and CCL9 (maximum at stage 2) was only transient. Although some differences were noticed between the transcriptional (Fig. 2B) and protein (Fig. 2C) expressions, RABV infection is characterized by a transient inflammatory response, with the expression of a majority of markers being rapidly down-regulated in the spinal cord and with a slight delay in the brain. Altogether, these data indicate that RABV triggers an inflammatory response in the NS, and suggest that regulatory mechanisms are set up in the course of the infection to reduce the RABV-induced inflammation of the NS.

### (2) RABV infection triggers low and transient inflammation in the liver

Previous reports have shown that an inflammation of NS as those caused by a neuro-trauma or a neuro-infection, [bladder cystitis caused by pseudo-rabies virus spinal cord infection (Jasmin *et al,* 1998) and inflammation of the liver caused by a neuro-trauma (Catania *et al,* 2009)], triggers an inflammation in the peripheral organs. Here, we investigated whether RABV which infects the spinal cord and the brain, and not the liver, can also trigger liver inflammation. Transcription of the 4 markers of inflammation, *TNFα, IL-6, IL-12, CXCL10,* and *CCL2* was followed in the liver of RABV-infected mice sacrificed at different stages of pathology (Fig. 3A). A low (maximum 10 fold increase), transient (maximum at stage 1) but significant induction was observed for the 4 markers in the liver of RABV-infected mice compared to NI. Then inflammation Ab arrays were performed and compared between the livers extracts of NI and RABV-infected mice exhibiting the first clinical signs (stage 1) or tetraplegia (stage 4), (Fig. 3B). The expression of most of the markers (29 out of 40) was decreased in infected mice or unchanged (7 markers) whereas the expression of only 4 markers was increased. Among them, the upregulation of the anti-inflammatory soluble receptors for the TNF, sTNFR1 and sTNFR2, was only transient (stage 1). The pattern of inflammation observed in the liver at both transcription and protein levels suggests that inflammation occurs in the liver of the RABV-infected mice very early after NS infection and is rapidly and efficiently down-regulated.

To check whether there is a correlation between the infection of the NS by the virus and the liver inflammation we analyzed the liver inflammation in a mouse strain in which spinal cord and brain are more rapidly colonized by RABV. The NesCre (^{+/-}) IFNAR (^{flox/flox}) mouse is a C57BI6 background mouse lacking the receptor of type I IFN in neural cells of ectodermal origin (Detje *et al,* 2009). In these mice RABV reaches the spinal cord and brain more rapidly than in WT mice leading to a more severe pathogenesis, a higher mortality rate and earlier death compared to WT mice (Fig. 8A) (Chopy D., 2011 in press). As shown in Fig. 8B, RABV-infected NesCre (^{+/-}) IFNAR (^{flox/flox}) mice exhibited higher *TNF*α, *CCL2, IL-1b* and *IL-6* expression in their spinal cord and their brain compared to RABV-infected C57BI6 mice (WT). Earlier presence of RABV and higher inflammatory response in the NS of NesCre(^{+/}-)IFNAR(^{flox/flox}) were associated with earlier and higher induction of *TNF*α, *CCL2* and *CXCL10* in their liver (Fig. 8C) compared to WT mice.

This suggests that inflammation of the liver is due to RABV accessibility and RABV-mediated inflammation and stress in the NS.

### (3) RABV-infected mice exhibit markers of the cholinergic pathway in the brain and the liver

The key role of TNFα in rabies (Camelo *et al,* 2000) and the tight regulation of TNFα expression in both the NS and the liver urged us to investigate whether the cholinergic signalling pathway was involved in rabies. Since phosphosphorylation of STAT3 and ERK1/2 and expression of SOCS3 are main markers of cholinergic signalling (Dajas-Bailador *et al,* 2002b; de Jonge *et al,* 2005), expression of these proteins were monitored by western blotting in extracts of brain (Fig. 4A and B) and liver (Fig. 4C and D) of RABV-infected WT and NesCre (^{+/-}) IFNAR (^{flox/flox}) mice sacrificed at different stages of RABV pathology. In the brain, phosphorylation of STAT3 and ERK1/2 was induced as the clinical signs progress, suggesting that cholinergic signalization is triggered in the brain during RABV infection. In addition STAT3 phosphorylation was dependent upon RABV accessibility to the nervous system since STAT3 activation occurs earlier in the brain of NesCre(^{+/-})IFNAR(^{flox/flox}) mice (Fig 4B right panel).

In the liver of RABV-infected WT mice, (Fig. 4C), STAT3 expression and STAT3 phosphorylation occurred at the two first stages of the pathogenesis (stage 1 and 2), and then decreased at later stages of the disease (stages 3 and 4). In contrast, SOCS3 and IkB expression increased as the clinical signs worsened. The transient phosphorylation of STAT3 was also observed in NesCre(^{+/-})IFNAR(^{flox/flox}) mice liver as soon as they showed clinical signs corresponding to stage 1 and 2 (Fig 4D). Nevertheless, these stages were reached at earlier time points in NesCre(^{+/-})IFNAR(^{flox/flox}) than in WT mice. This indicates that early phosphorylation of STAT3 is dependent on the speed with which the NS is infected (Fig. 4D). The transient phosphorylation of STAT3 at the early stages of the disease (stages 1 and 2) and the drastic control of inflammation in the liver at stage 4 (see Fig. 3) are consistent with the activation of a cholinergic pathway.

### (4) Post-exposure treatment of RABV-infected mice with mecamylamine, an α7nAchR antagonist, interrupts the cholinergic signalization pathway in the brain

To assay whether the cholinergic pathway was involved in RABV pathogenesis, WT mice were inoculated i.p. with mecamylamine (with 2mg/kg as previously described (Bhutada *et al,* 2010) at day 5 pi, corresponding roughly to stage 1 of the disease. N RABV gene expression was measured by qRT-PCR to evaluate RABV infection. As shown in Fig 5A, mock-treated mice and mice treated with mecamylamine exhibit similar RABV gene expression in their spinal cord and brain 8 days pi. Expression and phosphorylation of STAT3 and ERK1/2 were compared in the brain of RABV infected mice sacrificed 8dpi and of mock-treated or treated with mecamylamine 3 days earlier (5 day pi) (Fig. 5B). Despite similar brain infection, mecamylamine treatment reduces expression and phosphorylation of STAT3 and ERK1/2 to levels similar to those observed in the non-infected brain.

### (5) Post exposure mecamylamine treatment did not modify RABV-mediated brain inflammation

To investigate whether a day 5 pi mecamylamine treatment, which drastically altered the cholinergic signaling in the brain (see Fig. 5B), can modify RABV-mediated inflammatory response in the NS, qRT-PCR targeting *IL-6, CXCL10, IFN-*γ and *TNF*α transcript were performed in the spinal cord (Fig. 9A) and the brain (Fig. 9B) of RABV-infected mice treated or not with mecamylamine. In the brain, mecamylamine treatment did not modify significantly cytokines expression at transcriptional level. In the spinal cord, mecamylamine has no effect on IL6 or CXCL10 kinetic but slowed-down *IFN*-γ and *TNF*α transcription 8 days pi compared to mock treated animals. No difference was observed in inflammatory cytokines expression in the liver (data not shown).

These data indicate that mecamylamine has no effect on the control of brain inflammatory response.

### (6) Post exposure mecamylamine treatment prevents RABV-mediated spleen atrophy and restores immune cell subsets in the spleen of RABV infected mice

Cholinergic pathway triggers immuno-unresponsiveness through the modulation of the splenic nerve function (Huston *et al,* 2009). To test whether cholinergic pathway is involved RABV immune-unresponsiveness, numbers of thymocytes and splenocytes were compared in 5 and 8 day RABV-infected mice mock-treated or treated with mecamylamine (Fig. 6). As expected, RABV infection reduced significantly the number of thymocytes and splenocytes as soon as 5 day pi. In mice exhibiting similar viral transcript in their brain (Fig. 5A), mecamylamine treatment has no effect on the drop of thymocytes whereas it protects cellularity of the spleen. In absence of infection, mecamylamine injection increased the number of splenocytes indicating that interference with cholinergic pathway can enhance cell division in spleen.

Mecamylamine prevents the RABV-mediated disappearance of main subsets of spleen cells (Fig 6 B). This is the case of TCRαβ, CMH class II CD4, CD8 subsets as well as plasmocytes expressing IgG (double positive cells for IgG and CD19). Numbers of CD4 T cells expressing CD25 (the receptor of IL2) and numbers of CD3 expressing the marker of very early activation CD69 were also restored by a post exposure mecamylamine treatment.

Despite the increase of plasmocytes expressing IgG day 10 pi, no differences in RABV neutralizing antibodies were observed between RABV-infected mice treated or not treated mice (mock), meaning that mecamylamine treatment has no effect on RABV specific neutralizing antibodies (Table 1).

**Table 1: Antibody titres expressed in IU/ml as mean titer ± SD (n=6)**

| **Day pi** | **RABV + MOCK** | **RABV + MECA** | **Student'T test** |
|---|---|---|---|
| **7** | 6.8 ±1.2 | 6.6 ± 2.2 | p= 0.350 |
| **9** | 8.9 ± 3.7 | 9.5 ± 3.0 | p= 0.250 |
| **20** | 37.93 ± 33.2 | 21.7 ± 15.8 | p= 0.169 |

To note, mecamylamine treatment has no effect on the transcription of the glucocorticoid-inducible gene *Fkbp51* (Paakinaho *et al,* 2010). *Fkbp51* gene expression was induced during RABV infection and correlated with RABV access to the NS (Fig. 10A and 10B). Indeed, Fkbp51 expressions are similar in the brain of RABV infected WT and NesCre(^{+/-})IFNAR(^{flox/flox}) mice (Fig. 10C). This indicates that mecamylamine does not modify the RABV-mediated HPA axis stimulation.

These data indicate that a mecamylamine treatment diminishes the effect of RABV mediated immune-unresponsiveness in the periphery.

### (7) Post-exposure mecamylamine treatment protects RABV-infected mice from fatal issue

Neuronal Nitric Oxide synthase NOs, nNOS, is an enzyme constitutively expressed in discrete populations of neurons. It contributes to the regulation of NO that is implicated in several neuronal functions such as neurogenesis, neuro-hormone secretion, learning or memory (Zhou and Zhu, 2009). During RABV infection, *nNOS* expression is decreased (Fig 7A) and such a nNOS decrease can be taken as a marker of RABV pathogenesis (Koprowski *et al,* 1993). The nNOS transcription was compared in the brain of mock- and mecamylamine-treated mice in absence of infection (NI) or after infection (Fig. 7B). Mecamylamine treatment was found to block the nNOS decrease observed in the 8 day RABV-infected brain suggesting that mecamylamine treatment can counteract RABV pathogenesis.

Weight loss, clinical scores and survival curves were compared between RABV-infected mice treated or not with mecamylamine 6 days pi (Fig. 7C). Mecamylamine treatment does not induced obvious side effects such as weight loss or behavior modification in non-infected mice (data not shown). In contrast, a day 6 pi mecamylamine treatment interrupted the weight loss, decreases the severity of the clinical scores and spectacularly protected mice from fatal rabies. To note, mecamylamine treatment applied 1 or 3 days pi did not modify RABV pathogenesis compared to mock treated animals (data not shown). When RABV N protein transcripts were compared among survivors of the Mecamylamine treated mice and those of the mock treated group, day 20 pi, the mecamylamine-treated mice showed less viral transcripts than the mock-treated animals, suggesting that post exposure mecamylamine treatment contributes to RABV clearance off the brain (Fig 7D).

### (8) Post exposure treatment in the early stages of the infection

Post exposure treatment occurring in the early stages of the infection (day1 pi), when mice do not show any clinical signs, does not provide any benefit in survival (Figure 11), indicating that mecamylamine effect is not related to a putative antiviral effect of the drug, and strengthening that mecamylamine is a post exposure treatment of declared rabies.

In summary, in a model of experimental rabies in mice (Fig 1) in which CNS infection by the rabies virus (RABV) causes a severe immune unresponsiveness, the present application demonstrated that a post exposure treatment with mecamylamine, a blocker of the cholinergic signaling pathway (Fig 5), prevents spleen atrophy and disappearance of specific subsets of immune cells in the spleen (Fig 6). The mecamylamine treatment results in a clearance of infection off the brain (Fig 7) and confers protection of the infected animal against fatal rabies (Fig 7). Thus, mecamylamine appeared as the first post exposure treatment of declared rabies.

### CONCLUSION

In conclusion, RABV infection activates cholinergic pathway signaling in both the infected brain and the non-infected liver. A post-exposure treatment of RABV infected mice with an AchR antagonist interrupts the cholinergic signaling pathway and prevents apparition of pathogenesis markers in the infected brain. As a result, mice are protected against fatal rabies.

Inflammatory response in infected or injured organs is a serious threat for body homeostasis. NS has set up central neuro-endocrino regulatory pathways, involving HPA axis, and cholinergic anti-inflammatory pathways, to control excessive inflammatory reactions. Besides the obvious local benefit, these pathways can also lead to severe immune-unresponsiveness decreasing the host defenses.

It has been recognized several decades ago that infection of the brain with RABV, a neurotropic virus causing fatal encephalitis, triggers a severe immune-unresponsiveness (Camelo *et al,* 2001; Hirai *et al,* 1992; Kasempimolporn *et al,* 1997; Kasempimolporn *et al,* 2001; Perry *et al,* 1990; Torres-Anjel *et al,* 1988; Tshikuka *et al,* 1992; Wiktor *et al,* 1977a; Wiktor *et al,* 1977b). This immune impairment is characterized by a wasting syndrome accompanied with thymus atrophy, overall lymphoid depletion and impaired T cell functions. It involves the HPA axis, but also other yet unidentified pathways (Perry *et al,* 1990). Here, using a mouse model of experimental rabies we provided an important breakthrough demonstrating that, besides the HPA axis, RABV triggers also cholinergic anti-inflammatory pathway whose interruption with mecamylamine an antagonist of the cholinergic pathway was sufficient to prevent rabies death in the infected mice. Mecamylamine treatment restores spleen cellularity and nNOs expression in the infected NS. It counteracts weight loss, whereas it has no effect on central NS inflammation or on the HPA axis.

Mecamylamine was identified in the 1950s as an anti-hypertensive drug and is currently a promising molecule for the treatment of major depression (Lippiello *et al,* 2008). Mecamylamine is also used in research models for studying the anti-inflammatory cholinergic pathway (van Westerloo *et al,* 2006). Treatment of mice with α7nAchR non-competitive antagonist mecamylamine was preferred to experimental vagotomy, because vague nerve section would interrupt both efferent and afferent fibers, making difficult to bring evidence of the existence of a cholinergic reflex. Mecamylamine was also preferred to the use of α7nAchR deficient mice to prevent putative interference with RABV infection. Similar limitation could have been observed with mecamylamine treatment. Nevertheless and in contrast to a previous *in vitro* observation (Castellanos *et al,* 1997), no evidence that mecamylamine has an antiviral effect in RABV infected mice was found, since a day1 post exposure, mecamylamine treatment has no effect on RABV infection. Moreover, mecamylamine has been described as a non-competitive nAchR blocker that occupies the ion channel and not the nicotine binding site, making unlikely that mecamylamine competes with RABV glycoprotein for binding to muscle or brain AChR (Lafon, 2005).

The possibility that cholinergic pathway was activated in the central brain in the course of rabies was supported by the expression of markers of the cholinergic signalling such as STAT3, phosphorylated STAT3 and ERK1/2 and their disappearance (STAT3-P) or decrease (STAT3 and ERK1/2-P) after a mecamylamine treatment. The direct effect of mecamylamine on cholinergic signalization pathway in the brain is consistent with the capacity of the drug to cross the blood brain barrier (Lippiello *et al,* 2008).

Transient activation of STAT3 and STAT3-P in the early stages of the disease and the disappearance of these markers in later stages of infection, when liver inflammation is under control is fully consistent with the existence of a cholinergic anti-inflammatory reflex in the liver. In such a cholinergic anti-inflammatroy reflex, inflammation of the liver is sensed by the afferent fibres of the vagus nerve. This information is transported to and treated by centers present in the hypothalamus, the brain stem and frontal area of the brain. It leads to activation of efferent fibers of the vague nerve triggering either directly or with an intermediate relay in nervous ganglia, to the release of Ach. Ach increases the expression markers of the cholinergic anti-inflammatory pathway (STAT3, STAT3P, SOCS3) targeting the liver macrophage leading to a drastic control of inflammation in the liver. As soon as the inflammation is cleared off the liver, negative feed- back interrupts the activation of cholinergic pathway and the Ach release. This is consistent with the disappearance of the cholinergic markers in the liver at stage 3 and thereafter at stage 4 of the disease.

One can wonder how the non-infected liver can be inflamed during RABV infection. Even if the present study did not specifically address this point, we observed that liver inflammation was concomitant to the infection and inflammation of brain and unlikely the spinal cord. This reminds previous observation showing that RABV infection of spinal cord was not sufficient to trigger RABV-mediated immune unresponsiveness (Camelo *et al,* 2001). Making a parallel with previous report showing that an intracerebral inoculation of TNF-alpha or IL-1b was sufficient in absence of infection to trigger liver inflammation (Campbell *et al,* 2005; Campbell *et al,* 2008) it can be proposed in the case of rabies that the induction of proinflammatory cytokines (IL-6), chemokines (CCL2, CCL5), prostaglandins (COX-2) and inducible nitric oxide (iNOS) in the brain can cause liver inflammation. In contrast, the participation of brain IL-1β or TNF-α in liver inflammation in RABV infected animals is unlikely, since expression of these two cytokines as proteins remained unchanged in the course of RABV infection compared to those of not infected mice (see Fig 2C). Nevertheless, TNFα has been shown to play a key role in RABV immune-unresponsiveness with TNFα receptor deficient mice infected with RABV. These mice exhibit higher TNFα, IFNγ and IL-10 level in their brain, an increased splenocytes proliferation and a delayed mortality compared to WT (Camelo *et al,* 2001; Camelo *et al,* 2000). Alternatively, liver inflammation may be triggered through direct nerve interactions connecting brain to liver, as a pseudo rabies virus infection of the spinal cord does, triggering inflammation of the innervated non-infected bladder (Jasmin *et al,* 1998).

Treatment of RABV-infected mice with mecamylamine interrupts the spleen atrophy. This is consistent with the effect of mecamylamine on T cell function which increases CD4⁺T cell proliferation and cytokines (IFN-gamma, TNF-alpha and IL-6) production (Karimi *et al,* 2010) and with the capacity of α7nAchR antagonists to enhance cell division (De Rosa *et al,* 2009). Such an effect of mecamylamine on splenocytes division was indeed observed in the non-infected spleen (see Fig. 7). On the contrary to what was observed in spleen, a mecamylamine treatment has no effect on thymus atrophy, suggesting that other pathways, such as the HPA axis, may control the thymus atrophy. Alternatively, a 5-day or 6-day 6 pi mecamylamine treatment could occur too late to counteract thymus atrophy, an event shown to precede spleen atrophy (Torres-Anjel *et al,* 1988).

Mecamylamine treatment also restores nNOS expression in the brain, suggesting that the RABV-mediated activation of the cholinergic pathway could contribute to decrease nNOS expression in the infected NS. Restoration of nNOs expression is accompanied with reduced mortality and morbidity. This observation confirms that protection of nNOS pool, through the control nNOs can exert on the level of NO in the NS could be important for host recovery in encephalitis (Komatsu *et al,* 1999). It identifies nNOS expression in the infected NS as a good marker of RABV outcome.

Mecamylamine has no effect on RABV-mediated inflammation of the brain, indicating that other anti-inflammatory mechanisms are triggered rapidly after infection to limit brain inflammation. RABV mediated inflammation in the brain may be reduced through the intrinsic property of neurons to reduce inflammation and regulate microglial phenotype during infection or injury (Meuth *et al,* 2008) via the expression by neurons of receptors such as CD47, CD22, CD200 and by their ligands on glial cells (Griffiths *et al,* 2007; Hoek *et al,* 2000; Wright *et al,* 2000). The HPA axis which is activated during rabies (as previously described and as confirmed in this study showing that glucocorticoid inducible gene *Fkbp51* is induced by RABV infection) could also modulate the RABV induced brain inflammation (Rugstad, 1988) (Szabo *et al,* 1994).

Mecamylamine has no effect on the glucocorticoid inducible gene *Fkbp51,* suggesting that HPA axis remains intact in the mecamylamine treated mice. Despite the setting of HPA axis, mecamylamine treated mice were protected against fatal encephalitis, confirming that HPA axis and the mechanisms controlled by this axis may play a limited role in rabies death (Perry *et al,* 1990).

Although the modulation of the innate immune response through neuroimmunomodulatory pathway has been previously described for others pathogens such as Influenza virus (Jamieson *et al,* 2010), this study shows for the first time the implication of the cholinergic anti-inflammatory pathway in the CNS during virus infection. The possibility to prevent declared rabies by simply interrupting cholinergic anti-inflammatory pathway opens exciting insight for new postexposure treatment of rabies, and more generally of pathogen triggering an immune unresponsiveness.

### BIBLIOGRAPHY

Catania, A., C. Lonati, A. Sordi, and S. Gatti. 2009. Detrimental consequences of brain injury on peripheral cells. Brain Behav Immun 23:877-884.

Jasmin, L., G. Janni, H.J. Manz, and S.D. Rabkin. 1998. Activation of CNS circuits producing a neurogenic cystitis: evidence for centrally induced peripheral inflammation. J Neurosci 18:10016-10029.

Jasmin, L., E. Carstens, and A.I. Basbaum. 1997. Interneurons presynaptic to rat tail-flick motoneurons as mapped by transneuronal transport of pseudorabies virus: few have long ascending collaterals. Neuroscience 76:859-876.

Campbell, S.J., V.H. Perry, F.J. Pitossi, A.G. Butchart, M. Chertoff, S. Waters, R. Dempster, and D.C. Anthony. 2005. Central nervous system injury triggers hepatic CC and CXC chemokine expression that is associated with leukocyte mobilization and recruitment to both the central nervous system and the liver. Am J Pathol 166:1487-1497.

Campbell, S.J., I. Zahid, P. Losey, S. Law, Y. Jiang, M. Bilgen, N. van Rooijen, D. Morsali, A.E. Davis, and D.C. Anthony. 2008. Liver Kupffer cells control the magnitude of the inflammatory response in the injured brain and spinal cord. Neuropharmacology 55:780-787.

Tracey, K.J. 2009. Reflex control of immunity. Nature reviews. Immunology 9:418-428.

Johnston, G.R., and N.R. Webster. 2009. Cytokines and the immunomodulatory function of the vagus nerve. Br J Anaesth 102:453-462.

Szabo, C., C. Thiemermann, C.C. Wu, M. Perretti, and J.R. Vane. 1994. Attenuation of the induction of nitric oxide synthase by endogenous glucocorticoids accounts for endotoxin tolerance in vivo. Proceedings of the National Academy of Sciences of the United States of America 91:271-275.

Tait, A.S., C.L. Butts, and E.M. Sternberg. 2008. The role of glucocorticoids and progestins in inflammatory, autoimmune, and infectious disease. Journal of leukocyte biology 84:924-931.

Smets, L.A., G. Salomons, and J. van den Berg. 1999. Glucocorticoid induced apoptosis in leukemia. Advances in experimental medicine and biology 457:607-614.

Ulloa, L. 2005. The vagus nerve and the nicotinic anti-inflammatory pathway. Nat Rev Drug Discov 4:673-684.

Dajas-Bailador, F.A., A.J. Mogg, and S. Wonnacott. 2002. Intracellular Ca2+ signals evoked by stimulation of nicotinic acetylcholine receptors in SH-SY5Y cells: contribution of voltage-operated Ca2+ channels and Ca2+ stores. Journal of neurochemistry 81:606-614.

Dajas-Bailador, F.A., L. Soliakov, and S. Wonnacott. 2002. Nicotine activates the extracellular signal-regulated kinase 1/2 via the alpha7 nicotinic acetylcholine receptor and protein kinase A, in SH-SY5Y cells and hippocampal neurones. Journal of neurochemistry 80:520-530.

Pena, G., B. Cai, J. Liu, E.P. van der Zanden, E.A. Deitch, W.J. de Jonge, and L. Ulloa. 2010. Unphosphorylated STAT3 modulates alpha 7 nicotinic receptor signaling and cytokine production in sepsis. Eur J Immuno/ 40:2580-2589.

de Jonge, W.J., E.P. van der Zanden, F.O. The, M.F. Bijlsma, D.J. van Westerloo, R.J. Bennink, H.R. Berthoud, S. Uematsu, S. Akira, R.M. van den Wijngaard, and G.E. Boeckxstaens. 2005. Stimulation of the vagus nerve attenuates macrophage activation by activating the Jak2-STAT3 signaling pathway. Nature immunology 6:844-851.

Yoshikawa, H., M. Kurokawa, N. Ozaki, K. Nara, K. Atou, E. Takada, H. Kamochi, and N. Suzuki. 2006. Nicotine inhibits the production of proinflammatory mediators in human monocytes by suppression of I-kappaB phosphorylation and nuclear factor-kappaB transcriptional activity through nicotinic acetylcholine receptor alpha7. Clinical and experimental immunology 146:116-123.

Pavlov, V.A., and K.J. Tracey. 2006. Controlling inflammation: the cholinergic anti-inflammatory pathway. Biochem Soc Trans 34:1037-1040.

van Westerloo, D.J., I.A. Giebelen, S. Florquin, M.J. Bruno, G.J. Larosa, L. Ulloa, K.J. Tracey, and T. van der Poll. 2006. The vagus nerve and nicotinic receptors modulate experimental pancreatitis severity in mice. Gastroenterology 130:1822-1830.

Tracey, K.J., and E. Abraham. 1999. From mouse to man: or what have we learned about cytokine-based anti-inflammatory therapies? Shock 11:224-225.

Meisel, C., J.M. Schwab, K. Prass, A. Meisel, and U. Dirnagl. 2005. Central nervous system injury-induced immune deficiency syndrome. Nat Rev Neurosci 6:775-786.

Silverman, M.N., B.D. Pearce, C.A. Biron, and A.H. Miller. 2005. Immune modulation of the hypothalamic-pituitary-adrenal (HPA) axis during viral infection. Viral Immunol 18:41-78.

Roy, A., and D.C. Hooper. 2007. Lethal silver-haired bat rabies virus infection can be prevented by opening the blood-brain barrier. J Virol 81:7993-7998.

Camelo, S., M. Lafage, A. Galelli, and M. Lafon. 2001. Selective role for the p55 Kd TNF-alpha receptor in immune unresponsiveness induced by an acute viral encephalitis. J Neuroimmunol 113:95-108.

Jamieson, A.M., S. Yu, C.H. Annicelli, and R. Medzhitov. 2010. Influenza virus-induced glucocorticoids compromise innate host defense against a secondary bacterial infection. Cell Host Microbe 7:103-114.

Torres-Anjel, M.J., D. Volz, M.J. Torres, M. Turk, and J.G. Tshikuka. 1988. Failure to thrive, wasting syndrome, and immunodeficiency in rabies: a hypophyseal/hypothalamic/thymic axis effect of rabies virus. Rev Infect Dis 10 Suppl 4:S710-725.

Wiktor, T.J., P.C. Doherty, and H. Koprowski. 1977. In vitro evidence of cell-mediated immunity after exposure of mice to both live and inactivated rabies virus. Proc Natl Acad Sci U S A 74:334-338. Wiktor, T.J., P.C. Doherty, and H. Koprowski. 1977. Suppression of cell-mediated immunity by street rabies virus. J Exp Med 145:1617-1622.

Hirai, K., H. Kawano, K. Mifune, H. Fujii, A. Nishizono, A. Shichijo, and K. Mannen. 1992. Suppression of cell-mediated immunity by street rabies virus infection. Microbiol Immunol 36:1277-1290.

Kasempimolporn, S., W. Saengseesom, C. Mitmoonpitak, S. Akesowan, and V. Sitprija. 1997. Cell-mediated immunosuppression in mice by street rabies virus not restored by calcium ionophore or PMA. Asian Pac J Allergy Immunol 15:127-132.

Kasempimolporn, S., T. Tirawatnapong, W. Saengseesom, S. Nookhai, and V. Sitprija. 2001. Immunosuppression in rabies virus infection mediated by lymphocyte apoptosis. Jpn J Infect Dis 54:144-147.

Tshikuka, J.G., M.J. Torres-Anjel, D.C. Blenden, and S.C. Elliott. 1992. The microepidemiology of wasting syndrome, a common link to diarrheal disease, cancer, rabies, animal models of AIDS, and HIV-AIDS YHAIDS). The feline leukemia virus and rabies virus models. Ann N YAcad Sci 653:274-296.

Perry, L.L., J.D. Hotchkiss, and D.L. Lodmell. 1990. Murine susceptibility to street rabies virus is unrelated to induction of host lymphoid depletion. Journal of immunology 144:3552-3557.

Camelo, S., M. Lafage, and M. Lafon. 2000. Absence of the p55 Kd TNF-alpha receptor promotes survival in rabies virus acute encephalitis. J Neurovirol 6:507-518.

Detje, C.N., T. Meyer, H. Schmidt, D. Kreuz, J.K. Rose, I. Bechmann, M. Prinz, and U. Kalinke. 2009. Local type I IFN receptor signaling protects against virus spread within the central nervous system. J Immunol 182:2297-2304.

Bhutada, P.S., Y.R. Mundhada, K.U. Bansod, P.V. Dixit, S.N. Umathe, and D.R. Mundhada. 2010. Inhibitory influence of mecamylamine on the development and the expression of ethanol-induced locomotor sensitization in mice. Pharmacol Biochem Behav 96:266-273.

Paakinaho, V., H. Makkonen, T. Jaaskelainen, and J.J. Palvimo. 2010. Glucocorticoid receptor activates poised FKBP51 locus through long-distance interactions. Mol Endocrinol 24:511-525. Huston, J.M., M. Rosas-Ballina, X. Xue, O. Dowling, K. Ochani, M. Ochani, M.M. Yeboah, P.K. Chatterjee, K.J. Tracey, and C.N. Metz. 2009. Cholinergic neural signals to the spleen down-regulate leukocyte trafficking via CD11b. J Immunol 183:552-559.

Zhou, L., and D.Y. Zhu. 2009. Neuronal nitric oxide synthase: structure, subcellular localization, regulation, and clinical implications. Nitric oxide : biology and chemistry / official journal of the Nitric Oxide Society 20:223-230.

Koprowski, H., Y.M. Zheng, E. Heber-Katz, N. Fraser, L. Rorke, Z.F. Fu, C. Hanlon, and B. Dietzschold. 1993. In vivo expression of inducible nitric oxide synthase in experimentally induced neurologic diseases. Proc Natl Acad Sci U S A 90:3024-3027.

Lippiello, P.M., J.S. Beaver, G.J. Gatto, J.W. James, K.G. Jordan, V.M. Traina, J. Xie, and M. Bencherif. 2008. TC-5214 (S-(+)-mecamylamine): a neuronal nicotinic receptor modulator with antidepressant activity. CNS neuroscience & therapeutics 14:266-277.

Castellanos, J.E., D.R. Castaneda, A.E. Velandia, and H. Hurtado. 1997. Partial inhibition of the in vitro infection of adult mouse dorsal root ganglion neurons by rabies virus using nicotinic antagonists. Neurosci Lett 229:198-200.

Lafon, M. 2005. Rabies virus receptors. J Neurovirol 11:82-87.

Karimi, K., J. Bienenstock, L. Wang, and P. Forsythe. 2010. The vagus nerve modulates CD4+ T cell activity. Brain Behav Immun 24:316-323.

De Rosa, M.J., L. Dionisio, E. Agriello, C. Bouzat, and C. Esandi Mdel. 2009. Alpha 7 nicotinic acetylcholine receptor modulates lymphocyte activation. Life Sci 85:444-449.

Komatsu, T., D.D. Ireland, N. Chen, and C.S. Reiss. 1999. Neuronal expression of NOS-1 is required for host recovery from viral encephalitis. Virology 258:389-395.

Meuth, S.G., O.J. Simon, A. Grimm, N. Melzer, A.M. Herrmann, P. Spitzer, P. Landgraf, and H. Wiendl. 2008. CNS inflammation and neuronal degeneration is aggravated by impaired CD200-CD200R-mediated macrophage silencing. J Neuroimmunol 194:62-69.

Griffiths, M., J.W. Neal, and P. Gasque. 2007. Innate immunity and protective neuroinflammation: new emphasis on the role of neuroimmune regulatory proteins. Int Rev Neurobiol 82:29-55.

Hoek, R.M., S.R. Ruuls, C.A. Murphy, G.J. Wright, R. Goddard, S.M. Zurawski, B. Blom, M.E. Homola, W.J. Streit, M.H. Brown, A.N. Barclay, and J.D. Sedgwick. 2000. Down-regulation of the macrophage lineage through interaction with OX2 (CD200). Science 290:1768-1771.

Wright, G.J., M.J. Puklavec, A.C. Willis, R.M. Hoek, J.D. Sedgwick, M.H. Brown, and A.N. Barclay. 2000. Lymphoid/neuronal cell surface OX2 glycoprotein recognizes a novel receptor on macrophages implicated in the control of their function. Immunity 13:233-242.

Rugstad, H.E. 1988. Antiinflammatory and immunoregulatory effects of glucocorticoids: mode of action. Scandinavian journal of rheumatology. Supplement 76:257-264.

Lafon M (2011). Evasive strategies in rabies virus infection. Adv Virus Res 79: 33-53. Martinez-Arends A, Astoul E, Lafage M, Lafon M (1995). Activation of human tonsil lymphocytes by rabies virus nucleocapsid superantigen. Clin Immunol Immunopathol 77: 177-84.

Lockhart BP, Tordo N, Tsiang H (1992). Inhibition of rabies virus transcription in rat cortical neurons with the dissociative anesthetic ketamine. Antimicrob Agents Chemother 36: 1750-5.

Smith JS, Yager PA, Baer GM (1973). A rapid tissue culture test for determining rabies neutralizing antibody. Monogr Ser World Health Organ: 354-7.

## Claims

1. Mecamylamine or composition comprising mecamylamine, for use in the therapeutic treatment of an animal or human patient presenting with a disorder of the central nervous system, said animal or patient being suspected of having an infection by a pathogen which triggers an immune unresponsiveness.

2. Mecamylamine or composition comprising mecamylamine, for use to restore a therapeutic immune response in an animal or human patient presenting with a disorder of the central nervous system, said animal or patient being suspected of having an infection by a pathogen which triggers immune unresponsiveness.

3. Mecamylamine or composition for use according to claim 1 or 2, wherein said immune unresponsiveness triggered by the pathogen is mediated through the cholinergic anti-inflammatory patway.

4. Mecamylamine or composition comprising mecamylamine for use according to any one of claims 1 to 3, wherein said mecamylamine is selected from the group consisting of the (S)-(+)-mecamylamine, the (R)-(-)-mecamylamine, a combination of the (S)-(+)-mecamylamine and (R)-(-)-mecamylamine, salts of mecamylamine, such as mecamylamine hydrochloride, and mecamylamine analogues.

5. Mecamylamine or composition comprising mecamylamine for use according to any one of claims 1 to 4, wherein said pathogen is a neuroinvasive virus, in particular a neurotropic virus.

6. Mecamylamine or composition comprising mecamylamine for use according to any one of claims 1 to 5, wherein said pathogen is an immunosuppressive RNA virus.

7. Mecamylamine or composition comprising mecamylamine for use according to any one of claims 1 to 6, wherein said virus is selected from the group consisting of measles virus, rabies virus, New Castle disease virus, simian immunodeficiency virus (SIV) and human immunodeficiency virus (HIV).

8. Mecamylamine or composition comprising mecamylamine for use according to any one of claims 1 to 7, wherein said pathogen is a rabies virus.

9. Mecamylamine or composition comprising mecamylamine for use according to claim 8, wherein said mecamylamine or composition is administered once rabies clinical symptoms are observed.

10. Mecamylamine or composition comprising mecamylamine for use according to any one of claims 1 to 9 in a human patient.

11. Mecamylamine or composition comprising mecamylamine, for use to treat established rabies in a human patient.

12. Mecamylamine or composition comprising mecamylamine, for use to treat a human patient presenting with clinical symptoms of rabies.

13. The composition comprising mecamylamine for use according to any one of claims 1 to 12, further comprising inactive ingredients such as pharmaceutically suitable excipients and/or carriers and/or vehicles.

14. The composition comprising mecamylamine for use according to any one of claims 1 to 13, further comprising additional active ingredient(s), particularly other active ingredient(s) against said pathogen.

15. The composition comprising mecamylamine for use according to claim 14, wherein said additional ingredients are ketamine and/or midazolam and/or phenobarbital and/or ribavirin and/or amantadine

16. Mecamylamine or composition comprising mecamylamine for use according to any one of claims 1 to 15, wherein said mecamylamine or composition is administered through parenteral administration such as subcutaneous (s.c.), intradermal (i.d.), intramuscular (i.m.), intraperitoneal (i.p.) or intravenous (i.v.) injection, through oral administration or by mucosal administration.

17. Mecamylamine or composition comprising mecamylamine for use according to any one of claims 1 to 16, wherein mecamylamine is administered at an amount within a range from about 0.1 and 30 mg daily, in one or multiple administration dose(s).

18. Mecamylamine or composition comprising mecamylamine for use according to any one of claims 1 to 17, wherein the administration regimen encompasses administration of a unique dose of mecamylamine or said composition comprising mecamylamine.

19. Mecamylamine or composition comprising mecamylamine for use according to any one of claims 1 to 17, wherein the administration regimen encompasses administration of a repeated dose, either identical or different, of mecamylamine or said composition comprising mecamylamine.

20. Mecamylamine or composition comprising mecamylamine for use according to any one of claims 1 to 19, wherein mecamylamine is formulated in order to enable the administration of a therapeutic effective amount of mecamylamine to a human patient to provide its exposure to the brain of said human patient and the restoration of the immune response.

21. Mecamylamine or composition comprising mecamylamine for use according to any one of claims 1 to 20, in an administration as an adjunctive therapy or as a combination therapy, said therapy encompassing a treatment active against said pathogen or against the symptoms associated with the infection or against the symptoms associated with the CNS disorder(s).
